(19) Europäisches Patentamt
European Patent Office
Office européen des brevets

(11) Veröffentlichungsnummer: **0 147 850 B1**

(12) # EUROPÄISCHE PATENTSCHRIFT

(45) Veröffentlichungstag der Patentschrift: **14.06.89**

(51) Int. Cl.⁴: **C 07 D 295/12**, C 07 D 211/14, A 61 K 31/395

(21) Anmeldenummer: **84116359.5**

(22) Anmeldetag: **27.12.84**

(54) **Neue Phenylessigsäurederivate, diese Verbindungen enthaltende Arzneimittel und Verfahren zu ihrer Herstellung.**

(30) Priorität: **30.12.83 DE 3347565**

(43) Veröffentlichungstag der Anmeldung:
**10.07.85 Patentblatt 85/28**

(45) Bekanntmachung des Hinweises auf die Patenterteilung:
**14.06.89 Patentblatt 89/24**

(84) Benannte Vertragsstaaten:
**AT BE CH DE FR GB IT LI LU NL SE**

(56) Entgegenhaltungen:
**EP-A- 0 058 779**
**EP-A- 0 099 017**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Grell, Wolfgang, Dr., Amriswilstrasse 7, D-7950 Biberach 1 (DE)**
Erfinder: **Hurnaus, Rudolf, Dr., Silcherstrasse 19, D-7950 Biberach 1 (DE)**
Erfinder: **Griss, Gerhart, Dr., Verstorben (DE)**
Erfinder: **Sauter, Robert, Dr., Alber-Schweitzer-Weg 9, D-7958 Laupheim (DE)**
Erfinder: **Rupprecht, Eckhard, Dr., Riedbachstrasse 15, D-7960 Aulendorf-Tannhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 147 850 B1

ACTORUM AG

**Beschreibung**

In der EP-A-0 058 779 und in der EP-A-0 099 017 werden bereits Phenylessigsäurederivate beschrieben, welche eine Wirkung auf den Stoffwechsel, insbesondere jedoch eine blutzuckersenkende Wirkung, aufweisen.

Es wurde nun gefunden, das die neuen Phenylessigsäurederivate der allgemeinen Formel

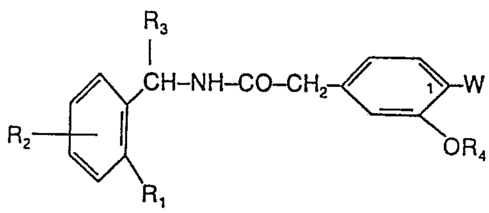

, (I)

die sich durch den Rest $-OR_4$ von den vorbeschriebenen Phenylessigsäurederivaten unterscheiden, wertvolle Eigenschaften besitzen. So weisen die Verbindungen der allgemeinen Formel I, in der $R_4$ eine Methyl-, Äthyl- oder Allylgruppe darstellt, überlegene pharmakologische Eigenschaften auf, insbesondere eine überlegene Wirkung auf den Stoffwechsel, vorzugsweise jedoch eine überlegene blutzuckersenkende Wirkung, und die Verbindungen der allgemeinen Formel I, in der $R_4$ ein Wasserstoffatom darstellt, stellen wertvolle Zwischenprodukte zur Herstellung der vorgenannten Verbindungen dar.

Gegenstand der vorliegenden Erfindung sind somit neue Phenylessigsäurederivate der obigen allgemeinen Formel I, deren Enantiomere und deren Salze, insbesondere deren physiologisch verträgliche Salze mit anorganischen oder organischen Säuren oder Basen, diese Verbindungen enthaltende Arzneimittel und Verfahren zu ihrer Herstellung.

In der obigen allgemeinen Formel I bedeuten

$R_1$ eine Pyrrolidino-, Piperidino-, Hexamethylenimino-, Methyl-pyrrolidino-, Dimethyl-pyrrolidino-, 2-Methyl-piperidino-, 3-Methyl-piperidino-, 4-Methylpiperidino-, 3,3-Dimethyl-piperidino-, cis-3,5-Dimethylpiperidino- oder trans-3,5-Dimethylpiperidinogruppe,

$R_2$ ein Wasserstoff- oder Halogenatom, eine Methyl- oder Methoxygruppe,

$R_3$ ein Wasserstoffatom, eine Alkylgruppe mit 1 bis 4 Kohlenstoffatomen, eine n-Pentyl- oder 3-Methyl-n-butylgruppe oder eine gegebenenfalls durch ein Halogenatom, eine Methyl- oder Methoxygruppe substituierte Phenylgruppe,

$R_4$ ein Wasserstoffatom, eine Methyl-, Äthyl- oder Allylgruppe und

W eine Methyl-, Hydroxymethyl-, Formyl-, Carboxy- oder Alkoxycarbonylgruppe mit insgesamt 2 bis 5 Kohlenstoffatomen, wobei der Alkylteil der Alkoxygruppe durch eine Phenylgruppe substituiert sein kann.

Für die bei der Definition der Reste $R_2$ bis $R_4$ und W eingangs erwähnten Bedeutungen kommen beispielsweise

für $R_2$ die des Wasserstoff-, Fluor-, Chlor- oder Bromatoms, der Methyl- oder Methoxygruppe,

für $R_3$ die des Wasserstoffatoms, der Methyl-, Äthyl-, n-Propyl-, Isopropyl-, n-Butyl-, Isobutyl-, sec.Butyl-, tert.Butyl-, n-Pentyl-, 3-Methyl-n-butyl-, Phenyl-, Fluorphenyl-, Chlorphenyl-, Bromphenyl-, Methylphenyl- oder Methoxyphenylgruppe, und

für W die der

Methyl-, Hydroxymethyl-, Formyl-, Carboxy-, Methoxycarbonyl-, Äthoxycarbonyl-, n-Propoxycarbonyl-, Isopropoxycarbonyl-, n-Butoxycarbonyl-, tert.Butoxycarbonyl-, Benzyloxycarbonyl-, 1-Phenyläthoxycarbonyl-, 2-Phenyläthoxycarbonyl- oder 3-Phenylpropoxycarbonylgruppe in Betracht.

Bevorzugte Verbindungen der obigen allgemeinen Formel I sind jedoch diejenigen, in denen

$R_3$ und $R_4$ wie vorstehend erwähnt definiert sind,

$R_1$ eine Pyrrolidino-, Piperidino-, 4-Methyl-piperidino-, 3-Methyl-piperidino-, 3,3-Dimethyl-piperidino-, 3,5-Dimethyl-piperidino- oder Hexamethyleniminogruppe,

$R_2$ ein Wasserstoff-, Fluor- oder Chloratom, eine Methyl- oder Methoxygruppe und

W eine Carboxy-, Methyl-, Hydroxymethyl-, Formyl- oder Benzyloxycarbonylgruppe oder eine Alkoxycarbonylgruppe mit insgesamt 2 bis 5 Kohlenstoffatomen darstellen,

insbesondere diejenigen Verbindungen, in denen

$R_1$ eine Piperidinogruppe,

$R_2$ ein Wasserstoff-, Fluor- oder Chloratom,

$R_3$ eine Methyl-, Äthyl-, n-Propyl-, n-Butyl-, Isobutyl-, n-Pentyl- oder Phenylgruppe,

$R_4$ eine Methyl- oder Äthylgruppe und

W eine Carboxy-, Methoxycarbonyl- oder Äthoxycarbonylgruppe darstellen,

deren Enantiomere und deren Salze, insbesondere deren physiologisch verträgliche Salze mit anorganischen oder organischen Säuren oder Basen.

Besonders bevorzugte Verbindungen der obigen allgemeinen Formel I sind diejenigen, in denen W die Carboxygruppe darstellt.

Erfindungsgemäß erhält man die neuen Verbindungen nach folgenden Verfahren:

a) Umsetzung eines Amins der allgemeinen Formel

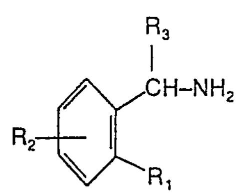

, (II)

in der

$R_1$ bis $R_3$ wie eingangs definiert sind, mit einer Carbonsäure der allgemeinen Formel

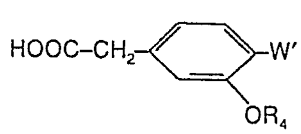

, (III)

in der

$R_4$ wie eingangs definiert ist und

W' die für W eingangs erwähnten Bedeutungen besitzt oder eine durch einen Schutzrest geschützte Carboxylgruppe darstellt,
oder mit deren gegebenenfalls im Reaktionsgemisch hergestellten reaktionsfähigen Derivaten und erforderlichenfalls anschließende Abspaltung eines verwendeten Schutzrestes.

Als reaktionsfähige Derivate einer Verbindung der allgemeinen Formel III kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen −25 und 250 °C, vorzugsweise jedoch bei Temperaturen zwischen −10 °C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Erforderlichenfalls wird die anschließende Abspaltung eines Schutzrestes vorzugsweise hydrolytisch durchgeführt, zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen −10 und 120 °C, z.B. bei Temperaturen zwischen Raumtemperatur und der Siedetemperatur des Reaktionsgemisches.

Ein als Schutzrest verwendeter tert.Butylrest kann auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure abgespalten werden.

Desweiteren kann ein als Schutzrest verwendeter Benzylrest auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid abgespalten werden.

b) Zur Herstellung einer Verbindung der allgemeinen Formel I, in der W eine Carboxygruppe darstellt:

Spaltung einer Verbindung der allgemeinen Formel

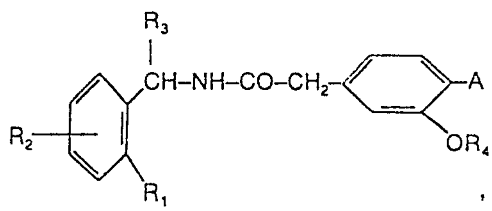

, (IV)

in der

$R_1$ bis $R_4$ wie eingangs definiert sind und

A eine durch Hydrolyse, Thermolyse oder Hydrogenolyse in eine Carboxygruppe überführbare Gruppe darstellt.

Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, die Tetrazolylgruppe, eine gegebenenfalls substituierte 1,3-Oxazol-2-yl- oder 1,3-Oxazolin-2-ylgruppe und

als thermolytisch abspaltbare Gruppen beispielsweise Ester mit tertiären Alkoholen, z.B. der tert.Butylester, und

als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen −10 und 120 °C, z.B. bei Temperaturen zwischen Raumtemperatur und der Siedetemperatur des Reaktionsgemisches, durchgeführt.

Bedeutet A in einer Verbindung der allgemeinen Formel IV eine Nitril- oder Aminocarbonylgruppe, so können diese Gruppen mittels 100%iger Phosphorsäure bei Temperaturen zwischen 100 und 180 °C, vorzugsweise bei Temperaturen zwischen 120 und 160 °C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50 °C in die Carboxygruppe übergeführt werden.

Bedeutet A in einer Verbindung der allgemeinen Formel IV beispielsweise die tert.Butyloxycarbonylgruppe, so kann die tert.Butylgruppe auch thermisch gegebenenfalls in einem inerten Lösungsmittel wie Methylenchlorid, Chloroform, Benzol, Toluol, Tetrahydrofuran oder Dioxan und

vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100 °C, abgespalten werden.

Bedeutet A in einer Verbindung der allgemeinen Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50 °C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert werden.

c) Zur Herstellung einer Verbindung der allgemeinen Formel I, in der $R_4$ ein Wasserstoffatom, eine Methyl- oder Äthylgruppe darstellt:

Umsetzung einer Verbindung der allgemeinen Formel

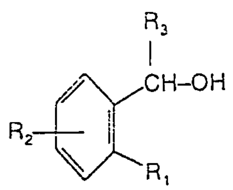

, (V)

in der

$R_1$ bis $R_3$ wie eingangs definiert sind, mit einer Verbindung der allgemeinen Formel

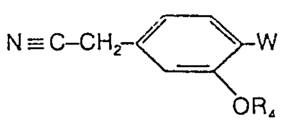

, (VI)

in der

$R_4$ und W wie eingangs definiert sind.

Die Umsetzung wird in Gegenwart einer starken Säure, welche gleichzeitig als Lösungsmittel dienen kann, vorzugsweise in konzentrierter Schwefelsäure, bei Temperaturen zwischen 0 und 150 °C, vorzugsweise bei Temperaturen zwischen 20 und 100 °C, durchgeführt.

Bedeutet in einer Verbindung der allgemeinen Formel VI $R_4$ eine Allylgruppe, so wird diese während der Umsetzung oder nach der Umsetzung bei Zugabe von Wasser abgespalten.

d) Zur Herstellung von Verbindungen der allgemeinen Formel I, in der $R_4$ ein Wasserstoffatom darstellt:

Abspaltung eines Schutzrestes von einer Verbindung der allgemeinen Formel

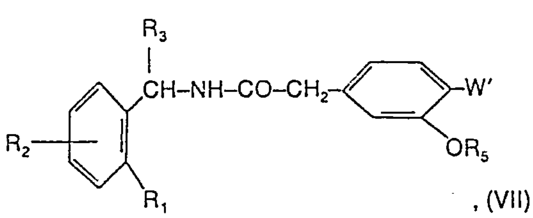

, (VII)

in der

$R_1$ bis $R_3$ wie eingangs definiert sind,

W' die für W eingangs erwähnten Bedeutungen besitzt oder eine in eine Carboxygruppe überführbare Gruppe darstellt und

$R_5$ eine Schutzgruppe für eine Hydroxygruppe darstellt.

Als eine in eine Carboxygruppe überführbare Gruppe kommt beispielsweise für W' eine Alkoxycarbonyl-, Aryloxycarbonyl-, Aralkoxycarbonyl-, Aminocarbonyl- oder Nitrilgruppe und als Schutzgruppe für $R_5$ eine Alkyl-, Aralkyl-, Trialkylsilyl- oder Acylgruppe, z.B. die Methyl-, Äthyl-, Propyl-, Benzyl-, Trimethylsilyl-, Acetyl- oder Propionylgruppe, in Betracht.

Die Abspaltung der oben genannten Schutzreste erfolgt je nach dem verwendeten Schutzrest entweder mittels Hydrolyse oder mittels Hydrogenolyse gegebenenfalls in einem geeigneten Lösungsmittel bei Temperaturen zwischen −78 und 250 °C. Beispielsweise erfolgt die Ätherspaltung in Gegenwart einer Säure wie Chlorwasserstoff, Bromwasserstoff, Schwefelsäure, Bortribromid, Aluminiumtrichlorid oder Pyridinhydrochlorid zweckmäßigerweise in einem geeigneten Lösungsmittel wie Methylenchlorid, Eisessig oder Wasser oder in deren Gemischen bei Temperaturen zwischen −78 und 250 °C. Hierbei wird die Ätherspaltung in Gegenwart einer Protonensäure zweckmäßigerweise bei Temperaturen zwischen 0 und 150 °C, vorzugsweise bei Temperaturen zwischen 50 und 150 °C, oder mit einer Lewis-Säure vorzugsweise in einem Lösungsmittel wie Methylenchlorid bei Temperaturen zwischen −78 und 20 °C, durchgeführt.

Beispielsweise erfolgt die Esterspaltung in Gegenwart einer Base wie wäßrigem Ammoniak, Natriumkarbonat, Kaliumcarbonat, Natrium- oder Kaliumhydroxid in einem Lösungsmittel wie Wasser, Wasser/Methanol, Wasser/Äthanol, Wasser/Tetrahydrofuran oder Wasser/Dioxan bei Temperaturen zwischen 50 und 100 °C, vorzugsweise bei Temperaturen des verwendeten Lösungsmittels.

Beispielsweise erfolgt die hydrogenolytische Abspaltung einer verwendeten Schutzgruppe wie der Benzylgruppe mit Wasserstoff in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid, vorzugsweise z.B. bei Raumtemperatur, und einem Wasserstoffdruck von 1 bis 5 bar.

e) Zur Herstellung von Verbindungen der allgemeinen Formel I, in der $R_4$ eine Methyl-, Äthyl- oder Allylgruppe darstellt:

Umsetzung einer Verbindung der allgemeinen Formel

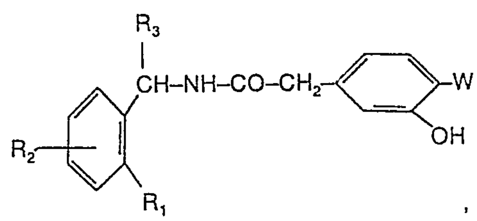

, (VIII)

in der

$R_1$ bis $R_3$ und W wie eingangs definiert sind, mit einer Verbindung der allgemeinen Formel

$$R_4'-X \quad (IX)$$

in der

$R_4'$ mit Ausnahme des Wasserstoffatoms wie eingangs definiert ist und

X eine nukleophil austauschbare Gruppe wie ein Halogenatom oder eine Sulfonyloxygruppe oder auch zusammen mit dem benachbarten Wasserstoffatom eine Diazogruppe, wenn $R_4'$ eine Methyl- oder Äthylgruppe darstellt, bedeuten und erforderlichenfalls anschließende Hydrolyse.

Die Umsetzung wird zweckmäßigerweise mit einem entsprechenden Halogenid, Anhydrid, Sulfonsäureester, Schwefelsäurediester oder Diazoalkan, z.B. mit Methyljodid, Dimethylsulfat, Äthylbromid, Diäthylsulfat, Propylbromid, Isopropylbromid, Allylbromid, p-Toluolsulfonsäure-äthylester, Methansulfonsäure-isopropylester oder Diazomethan, gegebenenfalls in Gegenwart einer Base wie Natriumhydrid, Kaliumcarbonat, Natriumhydroxid, Kalium-tert.butylat oder Triäthylamin oder im Falle der Acylierung mit einem Anhydrid auch in Gegenwart einer Säure wie Schwefelsäure vorzugsweise in einem geeigneten Lösungsmittel wie Aceton, Diäthyläther, Tetrahydrofuran, Dioxan, Pyridin oder Dimethylformamid bei Temperaturen zwischen 0 und 100 °C, vorzugsweise bei Temperaturen zwischen 20 und 50 °C, durchgeführt, wobei ein als Acylierungsmittel verwendetes Anhydrid gleichzeitig auch als Lösungsmittel verwendet werden kann.

Bedeutet W in einer Verbindung der allgemeinen Formel VIII eine Carboxy- oder Hydroxymethylgruppe, so kann diese in die entsprechende Esterverbindung übergeführt werden. Eine so erhaltene Verbindung wird erforderlichenfalls durch Spaltung der Estergruppe in die gewünschte Verbindung der allgemeinen Formel I übergeführt.

Die Spaltung der Estergruppe erfolgt hydrolytisch zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen −10 und 120 °C, z.B. bei Temperaturen zwischen Raumtemperatur und der Siedetemperatur des Reaktionsgemisches.

f) Reduktion einer Verbindung der allgemeinen Formel

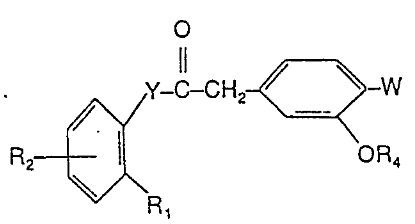
, (X)

in der

$R_1$, $R_2$, $R_4$ und W wie eingangs definiert sind und Y eine Gruppe der Formel

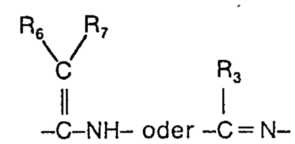
oder

darstellt, wobei $R_3$ wie eingangs definiert ist und $R_6$ und $R_7$ zusammen mit dem dazwischen liegenden Kohlenstoffatom eine Alkylidengruppe mit 1 bis 4 Kohlenstoffatomen darstellt.

Die Reduktion wird vorzugsweise mit Wasserstoff in Gegenwart eines Hydrierungskatalysators wie Palladium/Kohle oder Raney-Nickel in einem geeigneten Lösungsmittel wie Methanol, Äthanol, Isopropanol, Essigsäureäthylester, Dioxan, Tetrahydrofuran, Dimethylformamid, Benzol oder Benzol/Äthanol bei Temperaturen zwischen 0 und 100 °C, vorzugsweise jedoch bei Temperaturen zwischen 20 °C und 50 °C, und einem Wasserstoffdruck von 1 bis 5 bar durchgeführt. Bei Verwendung eines geeigneten chiralen Hydrierungskatalysators wie einem Metalligandenkomplex, z.B. einem Komplex aus µ,µ'-Dichlor-bis-[1,5-cyclooctadienrhodium] und (+)- oder (−) O,O-Isopropyliden-2,3-dihydroxy)-1,4-bis(diphenylphosphino)-butan (= DIOP), erfolgt die Wasserstoffanlagerung enantioselektiv. Desweiteren können bei der katalytischen Hydrierung andere Gruppen, z.B. eine Benzyloxygruppe zur Hydroxygruppe oder eine Formylgruppe zur Hydroxymethylgruppe, mitreduziert oder durch Wasserstoffatome, z.B. ein Halogenatom durch ein Wasserstoffatom, ersetzt werden.

Erhält man erfindungsgemäß

eine Verbindung der allgemeinen Formel I, in der $R_2$ ein Halogenatom und/oder $R_3$ eine Halogenphenylgruppe und/oder W eine Hydroxymethylgruppe, welche in eine Halogenmethylgruppe überführt wurde, bedeuten, so kann diese gewünschtenfalls mittels Enthalogenierung in eine entsprechende Verbindung der allgemeinen Formel I, in der $R_2$ ein Wasserstoffatom und/oder $R_3$ eine Phenylgruppe und/oder W die Methylgruppe bedeuten, übergeführt werden und/oder

eine Verbindung der allgemeinen Formel I, in der W die Carboxygruppe darstellt, so kann diese gewünschtenfalls mittels Veresterung in eine entsprechende Verbindung der allgemeinen Formel I, in der W eine Alkoxycarbonyl- oder Phenylalkoxycarbonylgruppe bedeutet, übergeführt werden, und/oder

eine Verbindung der allgemeinen Formel I, in der W eine Carboxy-, Alkoxycarbonyl- oder Phenylalkoxycarbonylgruppe darstellt, so kann diese mittels Reduktion in eine entsprechende Verbindung der allgemeinen Formel I, in der W eine Formyl- oder Hydroxymethylgruppe darstellt, übergeführt werden, und/oder

eine Verbindung der allgemeinen Formel I, in der W eine Hydroxymethylgruppe darstellt, so kann diese mittels Oxidation in eine entsprechende Verbindung der allgemeinen Formel I, in der W

eine Formyl- oder Carboxylgruppe darstellt, übergeführt werden, und/oder

eine Verbindung der allgemeinen Formel I, in der W die Carboxygruppe darstellt, so kann diese mittels Überführung in ein Sulfonsäurehydrazid und anschließende Disproportionierung in eine entsprechende Verbindung der allgemeinen Formel I, in der W die Formylgruppe darstellt, übergeführt werden, und/oder

eine racemische Verbindung der allgemeinen Formel I, in der $R_3$ mit Ausnahme des Wasserstoffatoms wie eingangs definiert ist, so kann diese über ihre diastereomeren Addukte, Komplexe oder Salze in die Enantiomeren aufgetrennt werden.

Die nachträgliche Enthalogenierung wird zweckmäßigerweise mittels katalytischer Hydrierung, z.B. an Palladium/Kohle, in einem geeigneten Lösungsmittel, z.B. Methanol, Äthanol, Tetrahydrofuran, Dioxan, Dimethylformamid oder Essigsäureäthylester, gegebenenfalls in Gegenwart einer Base wie Triäthylamin bei Temperaturen zwischen 20 und 100 °C, vorzugsweise bei 20 bis 50 °C, vorgenommen.

Die nachträgliche Veresterung wird zweckmäßigerweise in einem geeigneten Lösungsmittel, z.B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Thionylchlorid, Chlorameisensäureäthylester, Carbonyldiimidazol oder N,N'-Dicyclohexylcarbodiimid oder dessen Isoharnstoffäthern, gegebenenfalls in Gegenwart eines Reaktionsbeschleunigers wie Kupfer-chlorid, oder durch Umesterung, z.B. mit einem entsprechenden Kohlensäurediester, bei Temperaturen zwischen 0 und 100 °C, vorzugsweise jedoch bei Temperaturen zwischen 20 °C und der Siedetemperatur des betreffenden Lösungsmittels, durchgeführt.

Die nachträgliche Reduktion wird vorzugsweise mit einem Metallhydrid, z.B. mit einem komplexen Metallhydrid wie Lithiumaluminiumhydrid, Lithiumborhydrid oder Lithiumborhydrid/Borsäuretrimethylester, in einem geeigneten Lösungsmittel wie Diäthyläther, Tetrahydrofuran, Dioxan oder 1,2-Dimethoxyäthan bei Temperaturen zwischen 0 und 100 °C, vorzugsweise jedoch bei Temperaturen zwischen 20 °C und 60 °C, durchgeführt.

Die nachträgliche Oxidation eines Alkohols wird vorzugsweise mit einem Oxidationsmittel, z.B. mit Pyridinium-Chlorochromat oder Mangandioxid, in einem geeigneten Lösungsmittel wie Chloroform oder Methylenchlorid bei Temperaturen zwischen −10 und 50 °C, vorzugsweise jedoch bei Temperaturen zwischen 0 und 20 °C, durchgeführt.

Die nachträgliche Disproportionierung eines Sulfonsäurehydrazides, welches man durch Umsetzung eines entsprechenden Hydrazins mit einem entsprechenden reaktionsfähigen Carbonsäurederivat erhält, wird in Gegenwart einer Base wie Natriumkarbonat in einem Lösungsmittel wie Äthylenglykol bei Temperaturen zwischen 100 °C und 200 °C, vorzugsweise jedoch bei 160–170 °C, durchgeführt.

Die nachträgliche Racematspaltung wird vorzugsweise mittels Säulen- oder HPL-Chromatographie durch Bildung der diastereomeren Addukte oder Komplexe an einer chiralen Phase durchgeführt.

Die erhaltenen Verbindungen der allgemeinen Formel I lassen sich ferner in ihre Salze, insbesondere in ihre physiologisch verträglichen Salze mit anorganischen oder organischen Säuren oder auch Basen überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid, Kaliumhydroxid, Kalziumhydroxid, Cyclohexylamin, Äthanolamin, Diäthanolamin, Triäthanolamin, Äthylendiamin oder Lysin in Betracht.

Die als Ausgangsstoffe verwendeten Verbindungen der allgemeinen Formeln II bis X sind teilweise literaturbekannt bzw. erhält man nach an sich bekannten Verfahren.

So erhält man beispielsweise eine Verbindung der allgemeinen Formel II durch Reduktion eines entsprechenden Nitrils mit Lithiumaluminiumhydrid oder mit katalytisch angeregtem Wasserstoff, durch Umsetzung eines entsprechenden Nitrils mit einer entsprechenden Grignard- oder Lithium-Verbindung und anschließender Lithiumaluminiumhydrid-Reduktion oder anschließender Hydrolyse zum Ketimin, welches anschließend mit katalytisch angeregtem Wasserstoff, mit einem komplexen Metallhydrid oder mit nascierendem Wasserstoff reduziert wird, durch Hydrolyse bzw. durch Hydrazinolyse einer entsprechenden Phthalimidoverbindung, durch Umsetzung eines entsprechenden Ketons mit Ammoniumformiat und anschließender Hydrolyse bzw. mit einem Ammoniumsalz in Gegenwart von Natriumcyanoborhydrid, durch Reduktion eines entsprechenden Oxims mit Lithiumaluminiumhydrid, mit katalytisch angeregtem oder nascierendem Wasserstoff, durch Reduktion einer entsprechenden N-Benzyl- oder N-1-Phenyläthyl-Schiff'schen Base, z.B. mit einem komplexen Metallhydrid in Äther oder Tetrahydrofuran bei Temperaturen zwischen −78 °C und der Siedetemperatur des verwendeten Lösungsmittels und anschließender Abspaltung der Benzyl- oder 1-Phenyläthylgruppe mittels katalytischer Hydrierung, durch Ritter-Reaktion eines entsprechenden Alkohols mit Kaliumcyanid in Schwefelsäure, oder durch Hofmann-, Curtius-, Lossen- oder Schmidt-Abbau einer entsprechenden Verbindung.

Ein so erhaltenes Amin der allgemeinen Formel II mit einem chiralen Zentrum kann durch Racematspaltung, z.B. mittels fraktionierter Kristallisation der diastereomeren Salze mit optisch aktiven Säuren und anschließender Zerlegung der Salze oder durch Säulen- oder HPL-Chromatographie gegebenenfalls in Form seines Acylderivates oder durch Bildung von diastereomeren Verbindungen, deren Trennung und anschließende Spaltung in dessen Enantiomeren aufgetrennt werden.

Ferner kann ein optisch aktives Amin der allgemeinen Formel II auch durch enantio-selektive Reduktion eines entsprechenden Ketimins mittels komplexer Bor- oder Aluminiumhydride, in denen ein Teil der Hydridwasserstoffatome durch optisch aktive Alkoholatreste ersetzt ist, oder mittels Wasserstoff in Gegenwart eines geeigneten chiralen Hydrierungskatalysators bzw. analog ausgehend von einem entsprechenden N-Benzyl- oder N-(1-Phenäthyl)-ketimin oder von einem entsprechenden N-Acyl-ketimin bzw. Enamid und gegebenenfalls anschließende Abspaltung des Benzyl-, 1-Phenäthyl- oder Acylrestes hergestellt werden.

Ferner kann ein optisch aktives Amin der allgemeinen Formel II auch durch diastereo-selektive Reduktion eines entsprechenden am Stickstoffatom chiral substituierten Ketimins oder Hydrazons mittels komplexer oder auch nichtkomplexer Bor- oder Aluminiumhydride, in denen gegebenenfalls ein Teil der Hydridwasserstoffe durch entsprechende Alkoholat-, Phenolat- oder auch Alkyl-Reste ersetzt ist, oder mittels Wasserstoff in Gegenwart eines geeigneten Hydrierungskatalysators und gegebenenfalls anschließende Abspaltung des chiralen Hilfsrestes durch katalytische Hydrogenolyse oder Hydrolyse hergestellt werden.

Ferner kann ein optisch aktives Amin der allgemeinen Formel II auch durch diastereo-selektive Addition einer entsprechenden metallorganischen Verbindung, vorzugsweise einer Grignard- oder einer Lithiumverbindung, an ein entsprechendes am Stickstoffatom chiral substituiertes Aldimin, durch anschließende Hydrolyse und gegebenenfalls anschließende Abspaltung des chiralen Hilfsrestes durch katalytische Hydrogenolyse oder Hydrolyse hergestellt werden.

Die als Ausgangsstoffe verwendeten Verbindungen der allgemeinen Formeln IV, VII und VIII erhält man durch Umsetzung eines entsprechenden Amins mit einer entsprechenden Carbonsäure bzw. deren reaktiven Derivaten und gegebenenfalls anschließende Abspaltung eines verwendeten Schutzrestes.

Eine als Ausgangsstoff verwendete Verbindung der allgemeinen Formel V erhält man durch Reduktion einer entsprechenden Carbonylverbindung oder durch Umsetzung einer entsprechenden Carbonylverbindung mit einem entsprechenden Grignard- oder Lithium-Reagens.

Eine als Ausgangsstoff verwendete Verbindung der allgemeinen Formel X erhält man durch Acylierung einer entsprechenden Iminoverbindung oder deren metallorganischen Komplex mit einer entsprechenden Carbonsäure oder deren reaktiven Derivaten und gegebenenfalls anschließende Spaltung einer Estergruppe.

Wie bereits eingangs erwähnt, weisen die neuen Verbindungen der allgemeinen Formel I, in der $R_4$ eine Allylgruppe oder eine Alkylgruppe mit 1 bis 3 Kohlenstoffatomen darstellt, wertvolle pharmakologische Eigenschaften auf, nämlich eine Wirkung auf den Intermediärstoffwechsel, insbesondere jedoch eine blutzuckersenkende Wirkung.

Beispielsweise wurden die Verbindungen

A = 2-Methoxy-4-[N-(1-(2-piperidino-phenyl)-1-äthyl-amino-carbonylmethyl]-benzoesäure,

B = 2-Äthoxy-4-[N-(1-(2-piperidino-phenyl)-1-butyl)-amino-carbonylmethyl]-benzoesäure,

C = 2-Methoxy-4-[N-(α-phenyl-2-piperidino-benzyl)-aminocarbonyl-methyl]-benzoesäure,

D = 2-Äthoxy-4-[N-(α-phenyl-2-piperidino-benzyl)-aminocarbonyl-methyl]-benzoesäure,

E = (+)-2-Äthoxy-4-[N-(1-(2-piperidino-phenyl)-1-butyl)-aminocarbonyl-methyl]-benzoesäure,

F = 2-Äthoxy-4-[N-(1-(2-piperidino-phenyl)-1-äthyl)-amino-carbonylmethyl]-benzoesäure,

G = 2-Äthoxy-4-[N-(1-(2-pyrrolidino-phenyl)-1-butyl)-amino-carbonylmethyl]-benzoesäure-Natriumsalz,

H = 2-Äthoxy-4-[N-(1-(2-hexamethylenimino-phenyl)-1-butyl)-amino-carbonylmethyl]-benzoesäure,

I = 2-Methoxy-4-[N-(1-(2-piperidino-phenyl)-1-butyl)-amino-carbonylmethyl]-benzoesäure,

K = 2-Äthoxy-4-[N-(1-(2-piperidino-phenyl)-1-pentyl)-amino-carbonylmethyl]-benzoesäure und

L = 2-Äthoxy-4-[N-(4-methyl-α-phenyl-2-piperidino-benzyl)-amino-carbonylmethyl]-benzoesäure

auf ihre blutzuckersenkende Eigenschaft wie folgt untersucht:

Blutzuckersenkende Wirkung

Die blutzuckersenkende Wirkung der zu untersuchenden Substanzen wurde an weiblichen Ratten eigener Zucht mit dem Gewicht von 180–220 g geprüft, welche 24 Stunden vor Versuchsbeginn nüchtern gesetzt wurden. Die zu untersuchenden Substanzen wurden unmittelbar vor Versuchsbeginn in 1,5%iger Methylcellulose suspendiert und per Schlundsonde appliziert.

Die Blutentnahme erfolgte unmittelbar vor Substanzapplikation sowie 1, 2, 3 und 4 Stunden danach, jeweils aus dem retroorbitalen Venenplexus. Hiervon wurden jeweils 50 µl mit 0,5 ml 0,33 N Perchlorsäure enteiweißt und zentrifugiert. Im Überstand wurde Glukose nach der Hexokinase-Methode mit Hilfe eines Analysenphotometers bestimmt. Die statistische Auswertung erfolgte nach dem t-Test nach Student mit $p = 0,05$ als Signifikanzgrenze.

Die nachfolgende Tabelle enthält die gefundenen Werte in Prozent gegenüber Kontrolle:

| Substanz | 1 mg/kg | | | | 0,5 mg/kg | | | |
|---|---|---|---|---|---|---|---|---|
| | 1 | 2 | 3 | 4 h | 1 | 2 | 3 | 4 h |
| A | −37 | −46 | −23 | −14 | | | | |
| B | −38 | −49 | −38 | −33 | −43 | −36 | −34 | −35 |
| C | −38 | −41 | −38 | −34 | | | | |
| D | −42 | −54 | −37 | −34 | | | | |
| E | | | | | −40 | −39 | −36 | −36 |
| F | −44 | −44 | −40 | −30 | | | | |
| G | | | | | −40 | −33 | −30 | −17 |
| H | | | | | −42 | −34 | −18 | n. s. |
| I | | | | | −42 | −39 | −37 | −30 |
| K | | | | | −41 | −45 | −38 | −39 |
| L | −44 | −41 | −35 | −27 | | | | |

n. s. = statistisch nicht signifikant

Bei der Prüfung der Substanzen auf ihre blutzuckersenkende Wirkung konnten selbst bei einer Dosis von 10 mg/kg p.o. keine toxischen Nebenwirkungen beobachtet werden.

Die neuen Verbindungen sind praktisch untoxisch; beispielsweise verstarben nach einer einmaligen Dosis von 2000 mg/kg p.o. (Suspension in 1%iger Methylcellulose) der Substanzen B und D an jeweils 5 männliche und 5 weibliche Mäuse lediglich 1 Tier dieser Gruppe während der Nachbeobachtungszeit von 14 Tagen.

Aufgrund ihrer pharmakologischen Eigenschaften eignen sich die erfindungsgemäß hergestellten Verbindungen der allgemeinen Formel I, in der $R_4$ eine Methyl-, Äthyl- oder Allylgruppe darstellt, und deren physiologisch verträgliche Salze zur Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, Pulver oder Suspensionen einarbeiten. Die Einzeldosis am Erwachsenen beträgt hierbei 1–50 mg, vorzugsweise jedoch 2,5–20 mg, 1 oder 2 mal täglich.

Die nachfolgenden Beispiele sollen die Erfindung näher erläutern:

Beispiel 1

2-Äthoxy-4-[N-(1-(2-piperidino-phenyl-1-butyl)-aminocarbonylmethyl]-benzoesäure-äthylester

Zu einer Lösung von 1,84 g (7,9 mMol) 1-(2-Piperidino-phenyl)-1-butylamin in 19 ml Acetonitril gibt man nacheinander

2 g (7,9 mMol) 3-Äthoxy-4-äthoxycarbonyl-phenylessigsäure, 2,46 g (9,38 mMol) Triphenylphosphin, 1,7 ml (12,3 mMol) Triäthylamin und 0,76 ml (7,9 mMol) Tetrachlorkohlenstoff

und rührt zwei Tage bei Raumtemperatur. Anschließend dampft man im Vakuum ein und verteilt zwischen Äthylacetat und Wasser. Den organischen Extrakt trocknet und filtriert man und dampft ihn im Vakuum ein. Den Eindampfrückstand reinigt man durch Säulenchromatographie an Kieselgel (Toluol/Aceton = 5/1).

Ausbeute: 3 g (81% der Theorie),
Schmelzpunkt: 113–115 °C (Petroläther)

Ber.: C 72,07 H 8,21 N 6,00
Gef.: C 72,18 H 8,27 N 6,16

Analog Beispiel 1 wurden folgende Verbindungen erhalten:

a) 2-Methoxy-4-[N-(1-(2-piperidino-phenyl)-1-äthyl)-aminocarbonylmethyl]-benzoesäure-methylester

Hergestellt aus 1-(2-Piperidino-phenyl)-1-äthylamin und 3-Methoxy-4-methoxycarbonyl-phenylessigsäure.

Ausbeute: 78% der Theorie,
Schmelzpunkt: 82–85 °C
Ber.: C 70,22 H 7,37 N 6,82
Gef.: C 70,54 H 7,49 N 6,75

b) 2-Äthoxy-4-[N-(α-phenyl-2-piperidino-benzyl)-aminocarbonylmethyl]-benzoesäure-äthylester

Hergestellt aus α-Phenyl-2-piperidino-benzylamin und 3-Äthoxy-4-äthoxycarbonyl-phenylessigsäure.

Ausbeute: 77% der Theorie,
Schmelzpunkt: 149–151 °C
Ber.: C 74,37 H 7,25 N 5,60
Gef.: C 74,69 H 7,44 N 5,59

c) 2-Methoxy-4-[N-(α-phenyl-2-piperidino-benzyl)-aminocarbonylmethyl]-benzoesäure-methylester

Hergestellt aus α-Phenyl-2-piperidino-benzylamin und 3-Methoxy-4-methoxycarbonyl-phenylessigsäure.

Ausbeute: 65% der Theorie,
Schmelzpunkt: 189–190 °C
Ber.: C 73,70 H 6,83 N 5,93
Gef.: C 73,51 H 6,75 N 5,86

d) 2-Äthoxy-4-[N-(1-(2-piperidino-phenyl)-1-äthyl)-aminocarbonylmethyl]-benzoesäure-äthylester

Hergestellt aus 1-(2-Piperidino-phenyl)-1-äthylamin und 3-Äthoxy-4-äthoxycarbonyl-phenylessigsäure.

Ausbeute: 69% der Theorie,
Schmelzpunkt: 92–93 °C

Ber.: C 71,21 H 7,81 N 6,39
Gef.: C 71,29 H 8,03 N 6,58

e) 2-Äthoxy-4-[N-(1-(5-chlor-2-piperidino-phenyl)-1-propyl)-aminocarbonylmethyl]-benzoesäure-äthylester

Hergestellt aus 1-(5-Chlor-2-piperidino-phenyl)-1-propylamin und 3-Äthoxy-4-äthoxycarbonyl-phenyl-essigsäure.
Ausbeute: 80% der Theorie,
Schmelzpunkt: 110–112 °C
Ber.: C 66,58 H 7,24 N 5,75 Cl 7,28
Ger.: C 66,61 H 7,34 N 5,86 Cl 7,35

f) 2-Äthoxy-4-[N-(1-(2-piperidino-phenyl)-1-pentyl)-aminocarbonylmethyl]-benzoesäure-äthylester

Hergestellt aus 1-(2-Piperidino-phenyl)-1-pentylamin und 3-Äthoxy-4-äthoxycarbonyl-phenylessigsäure.
Ausbeute: 63% der Theorie,
Schmelzpunkt: 113–115 °C
Ber.: C 72,47 H 8,39 N 5,83
Gef.: C 72,66 H 8,26 N 5,99

g) 2-Äthoxy-4-[N-(1-(2-pyrrolidino-phenyl)-1-butyl)-aminocarbonylmethyl]-benzoesäure-äthylester

Hergestellt aus 1-(2-Pyrrolidino-phenyl)-1-butylamin und 3-Äthoxy-4-äthoxycarbonyl-phenylessigsäure.
Ausbeute: 50% der Theorie,
Schmelzpunkt: 85–87 °C
Ber.: C 71,65 H 8,02 N 6,19
Gef.: C 71,90 H 8,37 N 6,34

h) 2-Äthoxy-4-[N-(1-(2-(4-methyl-piperidino)-phenyl)-1-butyl)-aminocarbonyl-methyl]-benzoesäure-äthylester

Hergestellt aus 1-(2-(4-Methyl-piperidino)-phenyl)-1-butylamin und 3-Äthoxy-4-äthoxycarbonyl-phenylessigsäure.
Ausbeute: 44% der Theorie,
Schmelzpunkt: 127–128 °C
Ber.: C 72,47 H 8,39 N 5,83
Gef.: C 72,20 H 8,23 N 5,69

i) 2-Äthoxy-4-[N-(1-(2-hexamethylenimino-phenyl)-1-butyl)-aminocarbonylmethyl]-benzoesäure-äthylester

Hergestellt aus 1-(2-Hexamethylenimino-phenyl)-1-butylamin und 3-Äthoxy-4-äthoxycarbonyl-phenylessigsäure.
Ausbeute: 44% der Theorie,
Schmelzpunkt: 97–100 °C
Ber.: C 72,47 H 8,39 N 5,83
Gef.: C 72,41 H 8,50 N 5,66

k) 2-Äthoxy-4-[N-(1-(4-methyl-2-piperidino-phenyl)-1-butyl)-aminocarbonylmethyl]-benzoesäure-äthylester

Hergestellt aus 1-(4-Methyl-2-piperidino-phenyl)-1-butylamin und 3-Äthoxy-4-äthoxycarbonyl-phenylessigsäure.
Ausbeute: 68% der Theorie,
Schmelzpunkt: 113–114 °C
Ber.: C 72,47 H 8,39 N 5,83
Gef.: C 72,36 H 8,31 N 5,91

l) 2-Äthoxy-4-[N-(1-(6-methyl-2-piperidino-phenyl)-1-butyl)-aminocarbonylmethyl]-benzoesäure-äthylester

Hergestellt aus 1-(6-Methyl-2-piperidino-phenyl)-1-butylamin und 3-Äthoxy-4-äthoxycarbonyl-phenylessigsäure.
Ausbeute: 62% der Theorie,
Schmelzpunkt: < 20 °C
Ber.: C 72,47 H 8,39 N 5,83
Gef.: C 72,30 H 8,50 N 5,72

m) 2-Äthoxy-4-[N-(1-(6-chlor-2-piperidino-phenyl)-1-butyl)-aminocarbonylmethyl]-benzoesäure-äthylester

Hergestellt aus 1-(6-Chlor-2-piperidino-phenyl)-1-butylamin und 3-Äthoxy-4-äthoxycarbonyl-phenylessigsäure.
Ausbeute: 85% der Theorie,
Schmelzpunkt: < 20 °C
Ber.: C 67,12 H 7,44 N 5,50 Cl 7,08
Gef.: C 67,60 H 7,77 N 5,95 Cl 7,24

n) 2-Äthoxy-4-[N-(1-(4-methoxy-2-piperidino-phenyl)-1-butyl)-amino-carbonylmethyl]-benzoesäure-äthylester

Hergestellt aus 1-(4-Methoxy-2-piperidino-phenyl)-1-butylamin und 3-Äthoxy-4-äthoxycarbonyl-phenylessigsäure.
Ausbeute: 65% der Theorie,
Schmelzpunkt: 109–110 °C
Ber.: Molpeak m/e = 496
Gef.: Molpeak m/e = 496

o) 2-Äthoxy-4-[N-(1-(5-methoxy-2-piperidino-phenyl)-1-butyl)-amino-carbonylmethyl]-benzoesäure-äthylester

Hergestellt aus 1-(5-Methoxy-2-piperidino-phenyl)-1-butylamin und 3-Äthoxy-4-äthoxycarbonyl-phenylessigsäure.
Ausbeute: 31% der Theorie,
Schmelzpunkt: 117–120 °C
Ber.: Molpeak m/e = 496
Gef.: Molpeak m/e = 496

p) 2-Hydroxy-4-[N-(1-(2-piperidino-phenyl)-1-butyl)-aminocarbonylmethyl]-benzoesäure-äthylester

Hergestellt aus 1-(2-Piperidino-phenyl)-1-butylamin und 4-Äthoxycarbonyl-3-hydroxy-phenylessigsäure.
Ausbeute: 46% der Theorie,
Schmelzpunkt: 133–134 °C
Ber.: C 71,21 H 7,81 N 6,39
Gef.: C 71,08 H 7,91 N 6,45

q) 2-Methoxy-4-[N-(1-(2-piperidino-phenyl)-1-butyl)-aminocarbonylmethyl]-benzoesäure-methylester

Hergestellt aus 1-(2-Piperidino-phenyl)-1-butylamin und 3-Methoxy-4-methoxycarbonyl-phenylessigsäure.

Ausbeute: 67% der Theorie,
Schmelzpunkt: 128–131 °C

| | | | |
|---|---|---|---|
| Ber.: | C 71,21 | H 7,81 | N 6,39 |
| Gef.: | C 71,46 | H 7,80 | N 6,07 |

r) 2-Äthoxy-4-[N-(5-chlor-2-piperidino-benzyl)-aminocarbonylmethyl]-benzoesäure-äthylester

Hergestellt aus 5-Chlor-2-piperidino-benzylamin und 3-Äthoxy-4-äthoxycarbonyl-phenylessigsäure.
Ausbeute: 65% der Theorie,
Schmelzpunkt: 106–108 °C

| | | | | |
|---|---|---|---|---|
| Ber.: | C 65,41 | H 6,81 | N 6,10 | Cl 7,73 |
| Gef.: | C 65,81 | H 6,89 | N 6,11 | Cl 7,62 |

s) (−)-2-Äthoxy-4-[N-(α-phenyl-2-piperidino-benzyl)-aminocarbonylmethyl]-benzoesäure-äthylester

Hergestellt aus (−)-α-Phenyl-2-piperidino-benzylamin und 3-Äthoxy-4-äthoxycarbonyl-phenylessigsäure.
Ausbeute: 87% der Theorie,
Schmelzpunkt: 110–111 °C
Ber.: Molpeak m/e = 500
Gef.: Molpeak m/e = 500
Spezifische Drehung: $[\alpha]_D^{20} = -6{,}3°$ (c = 1, Methanol)

t) 2-Äthoxy-4-[N-(6-methyl-α-phenyl-2-piperidino-benzyl)-aminocarbonylmethyl]-benzoesäure-äthylester

Hergestellt aus 6-Methyl-α-phenyl-benzylamin und 3-Äthoxy-4-äthoxycarbonyl-phenylessigsäure.
Ausbeute: 39% der Theorie,
Schmelzpunkt: <20 °C

| | | | |
|---|---|---|---|
| Ber.: | C 74,68 | H 7,44 | N 5,44 |
| Gef.: | C 74,81 | H 7,56 | N 5,32 |

u) 2-Äthoxy-4-[N-(α-(4-methyl-phenyl)-2-piperidino-benzyl)-aminocarbonylmethyl]-benzoesäure-äthylester

Hergestellt aus α-(4-Methyl-phenyl)-2-piperidino-benzylamin und 3-Äthoxy-4-äthoxycarbonyl-phenylessigsäure.
Ausbeute: 34% der Theorie,
Schmelzpunkt: 150–152 °C

| | | | |
|---|---|---|---|
| Ber.: | C 74,68 | H 7,44 | N 5,44 |
| Gef.: | C 74,71 | H 7,51 | N 5,29 |

v) 2-Äthoxy-4-[N-(α-phenyl-2-pyrrolidino-benzyl)-aminocarbonylmethyl]-benzoesäure-äthylester

Hergestellt aus α-Phenyl-2-pyrrolidino-benzylamin und 3-Äthoxy-4-äthoxycarbonyl-phenylessigsäure.
Ausbeute: 45% der Theorie,
Schmelzpunkt: 85–87 °C

| | | | |
|---|---|---|---|
| Ber.: | C 74,05 | H 7,04 | N 5,76 |
| Gef.: | C 73,95 | H 7,07 | N 5,70 |

w) 2-Methoxy-4-[N-(2-hexamethylenimino-α-phenyl-benzyl)-aminocarbonylmethyl]-benzoesäure-methylester

Hergestellt aus 2-Hexamethylenimino-α-phenyl-benzylamin und 3-Methoxy-4-methoxycarbonyl-phenylessigsäure.
Ausbeute: 45% der Theorie,
Schmelzpunkt: 181–183 °C

| | | | |
|---|---|---|---|
| Ber.: | C 74,05 | H 7,04 | N 5,74 |
| Gef.: | C 74,09 | H 6,62 | N 5,74 |

x) 2-Äthoxy-4-[N-(2-hexamethylenimino-α-phenyl-benzyl)-aminocarbonylmethyl]-benzoesäure-äthylester

Hergestellt aus 2-Hexamethylenimino-α-phenyl-benzylamin und 3-Äthoxy-4-äthoxycarbonyl-phenylessigsäure.
Ausbeute: 41% der Theorie,
Schmelzpunkt: 140–141 °C

| | | | |
|---|---|---|---|
| Ber.: | C 74,68 | H 7,44 | N 5,44 |
| Gef.: | C 74,46 | H 7,62 | N 5,45 |

y) 2-Äthoxy-4-[N-(1-(2-piperidino-phenyl)-1-butyl)-aminocarbonylmethyl]-toluol

Hergestellt aus 1-(2-Piperidino-phenyl)-1-butylamin und 3-Äthoxy-4-methyl-phenylessigsäure.
Ausbeute: 55% der Theorie,
Schmelzpunkt: 107–108 °C

| | | | |
|---|---|---|---|
| Ber.: | C 76,43 | H 8,88 | N 6,86 |
| Gef.: | C 76,38 | H 8,99 | N 6,97 |

Beispiel 2

(+)-2-Äthoxy-4-[N-(1-(2-piperidino-phenyl)-1-butyl)-aminocarbonylmethyl]-benzoesäure-äthylester

0,90 g (3,57 mMol) 3-Äthoxy-4-äthoxycarbonyl-phenylessigsäure und 0,61 g (3,73 mMol) N,N'-Carbonyldiimidazol werden in 9 ml absolutem Tetrahydrofuran 5 Stunden am Rückfluß erhitzt. Dann wird die von Lösung 0,85 g (3,67 mMol) (+)-1-(2-Piperidino-phenyl)-1-butylamin (ee = 94,2) in 9 ml absolutem Tetrahydrofuran zugesetzt und 3 Stunden am Rückfluß erhitzt. Man dampft im Vakuum ein und verteilt den Eindampfrückstand zwischen Chloroform und Wasser. Die organische Phase wird getrocknet, filtriert und im Vakuum eingedampft. Den eingedampften Extrakt reinigt man säulenchromatographisch an Kieselgel (Toluol/Aceton = 10/1).
Ausbeute: 0,85 g (51,2% der Theorie),
Schmelzpunkt: 118–119 °C (Petroläther/Toluol = 50/2)

| | | | |
|---|---|---|---|
| Ber.: | C 72,07 | H 8,21 | N 6,00 |
| Gef.: | C 72,43 | H 8,34 | N 6,00 |

Spezifische Drehung: $[\alpha]_D^{20} = +7{,}1°$ (c = 1,06 in Methanol)

Beispiel 3

2-Äthoxy-4-[N-(α-phenyl-2-piperidino-benzyl)-aminocarbonylmethyl]-benzoesäure-äthylester

Zu einem Gemisch von 30 ml konzentrierter Schwefelsäure und 30 ml o-Dichlorbenzol tropft man bei 23–25 °C eine Lösung von 4,7 g (20 mMol) 2-Äthoxy-4-cyanomethyl-benzoesäure-äthylester und 5,3 g (20 mMol) α-Phenyl-2-piperidino-benzylalkohol in 30 ml o-Dichlorbenzol. Man rührt 2 Stunden bei Raumtemperatur nach. Anschließend

trennt man die o-Dichlorbenzol-Phase ab und gibt den Rückstand auf Eis. Nach Alkalisieren mit Sodalösung wird mit Chloroform extrahiert. Die Extrakte werden über Magnesiumsulfat getrocknet und eingeengt. Der Rückstand wird mit Petroläther (30–60°) verrieben, abfiltriert und an Kieselgel (Toluol/Äthylacetat = 5/1) durch Säulenchromatographie gereinigt.
Ausbeute: 5,6 g (56% der Theorie),
Schmelzpunkt: 150–151 °C

| | | | |
|---|---|---|---|
| Ber.: | C 74,37 | H 7,25 | N 5,60 |
| Gef.: | C 74,59 | H 7,41 | N 5,45 |

Analog Beispiel 3 wurden folgende Verbindungen erhalten:

a) 2-Methoxy-4-[N-(α-phenyl-2-piperidino-benzyl)-aminocarbonylmethyl]-benzoesäure-methylester

Hergestellt aus α-Phenyl-2-piperidino-benzylalkohol und 4-Cyanomethyl-2-methoxy-benzoesäure-methylester.
Ausbeute: 34% der Theorie,
Schmelzpunkt: 189–191 °C

| | | | |
|---|---|---|---|
| Ber.: | C 73,70 | H 6,83 | N 5,93 |
| Gef.: | C 73,63 | H 7,05 | N 5,95 |

b) 2-Äthoxy-4-[N-(α-phenyl-2-piperidino-benzyl)-aminocarbonylmethyl]-benzoesäure

Hergestellt aus α-Phenyl-2-piperidino-benzylalkohol und 2-Äthoxy-4-cyanomethyl-benzoesäure. Extraktion bei pH 5.
Ausbeute: 47% der Theorie,
Schmelzpunkt: 154–155 °C

| | | | |
|---|---|---|---|
| Ber.: | C 73,70 | H 6,83 | N 5,93 |
| Gef.: | C 73,61 | H 6,72 | N 5,65 |

c) 2-Methoxy-4-[N-(α-phenyl-2-piperidino-benzyl]-aminocarbonylmethyl]-benzoesäure

Hergestellt aus α-Phenyl-2-piperidino-benzylalkohol und 4-Cyanomethyl-2-methoxy-benzoesäure. Extraktion bei pH 5.
Ausbeute: 30% der Theorie,
Schmelzpunkt: 202–204 °C

| | | | |
|---|---|---|---|
| Ber.: | C 73,34 | H 6,59 | N 6,11 |
| Gef.: | C 73,17 | H 6,41 | N 6,05 |

d) 2-Äthoxy-4-[N-(1-(2-piperidino-phenyl)-1-butyl)-aminocarbonylmethyl]-benzoesäure-äthylester

Hergestellt aus 1-(2-Piperidino-phenyl)-1-butanol und 2-Äthoxy-4-cyanomethyl-benzoesäure-äthylester.
Ausbeute: 5% der Theorie,
Schmelzpunkt: 112–114 °C,

| | | | |
|---|---|---|---|
| Ber.: | C 72,07 | H 8,21 | N 6,00 |
| Gef.: | C 72,29 | H 8,46 | N 6,31 |

e) 2-Methoxy-4-[N-(1-(2-piperidino-phenyl)-1-äthyl)-aminocarbonylmethyl]-benzoesäure-methylester

Hergestellt aus 1-(2-Piperidino-phenyl)-1-äthanol und 4-Cyanomethyl-2-methoxy-benzoesäure-methylester.
Ausbeute: 18% der Theorie,
Schmelzpunkt: 83–85 °C.

| | | | |
|---|---|---|---|
| Ber.: | C 70,22 | H 7,37 | N 6,82 |
| Gef.: | C 70,60 | H 7,29 | N 6,97 |

f) 2-Methoxy-4-[N-(1-(2-piperidino-phenyl)-1-äthyl)-aminocarbonylmethyl]-benzoesäure

Hergestellt aus 1-(2-Piperidino-phenyl)-1-äthanol und 4-Cyanomethyl-2-methoxy-benzoesäure. Extraktion bei pH 5,5.
Ausbeute: 21% der Theorie,
Schmelzpunkt: 118–120 °C.
Ber.: m/e = 396
Gef.: m/e = 396

g) 2-Äthoxy-4-[N-(4-methyl-α-phenyl-2-piperidino-benzyl)-aminocarbonylmethyl]-benzoesäure-äthylester

Hergestellt aus 4-Methyl-α-phenyl-2-piperidino-benzylalkohol und 2-Äthoxy-4-cyanomethyl-benzoesäure-äthylester.
Ausbeute: 46% der Theorie,
Schmelzpunkt: 124–125 °C

| | | | |
|---|---|---|---|
| Ber.: | C 74,68 | H 7,44 | N 5,44 |
| Gef.: | C 74,81 | H 7,56 | N 5,32 |

h) 2-Methoxy-4-[N-(α-(4-chlor-phenyl)-2-piperidino-benzyl)-aminocarbonylmethyl)-benzoesäure-methylester

Hergestellt aus α-(4-Chlorphenyl)-2-piperidino-benzylalkohol und 2-Methoxy-4-cyanomethyl-benzoesäure-methylester.
Ausbeute: 47% der Theorie,
Schmelzpunkt: 176–178 °C

| | | | | |
|---|---|---|---|---|
| Ber.: | C 68,70 | H 6,17 | N 5,53 | Cl 6,99 |
| Gef.: | C 69,05 | H 5,93 | N 5,76 | Cl 7,10 |

i) 2-Hydroxy-4-[N-(α-phenyl-2-piperidino-benzyl)-aminocarbonylmethyl]-benzoesäure-äthylester

Hergestellt aus α-Phenyl-2-piperidino-benzylalkohol und 4-Cyanomethyl-2-hydroxy-benzoesäure-äthylester.
Ausbeute: 78% der Theorie,
Schmelzpunkt: 172–174 °C

| | | | |
|---|---|---|---|
| Ber.: | C 73,70 | H 6,83 | N 5,93 |
| Gef.: | C 73,80 | H 6,81 | N 5,83 |

Beispiel 4

2-Äthoxy-4-[N-(1-(2-piperidino-phenyl)-1-butyl)-aminocarbonylmethyl]-benzoesäure

Man rührt das Gemisch aus 2 g (4,3 mMol) 2-Äthoxy-4-[N-(1-(2-piperidino-phenyl)-1-butyl)-aminocarbonylmethyl)-benzoesäure-äthylester und 5,3 ml 1N-Natronlauge in 20 ml Äthanol 3 Stunden bei 60 °C, neutralisiert dann mit 5,3 ml 1N-Salzsäure und dampft im Vakuum das Äthanol ab. Man verteilt zwischen Äthylacetat und Wasser; den organischen Extrakt trocknet und filtriert man und dampft ihn im Vakuum ein. Den Eindampfrückstand kristallisiert man aus Petroläther unter Zusatz von Äthanol.
Ausbeute: 1,3 g (69% der Theorie),
Schmelzpunkt: 88–90 °C

Ber.: C 71,21 H 7,81 N 6,39
Gef.: C 71,62 H 7,73 N 6,54

Analog Beispiel 4 wurden folgende Verbindungen erhalten:

a) 2-Methoxy-4-[N-(1-(2-piperidino-phenyl)-1-äthyl)-aminocarbonylmethyl]-benzoesäure x 0,67 $H_2O$

Hergestellt aus 2-Methoxy-4-[N-(1-(2-piperidino-phenyl)-1-äthyl)-amino-carbonylmethyl]-benzoesäure-methylester.
Ausbeute: 60% der Theorie,
Schmelzpunkt: 116–120 °C
Ber.: C 67,62 H 7,07 N 6,85
Gef.: C 67,60 H 6,87 N 6,55

b) 2-Äthoxy-4-[N-(α-phenyl-2-piperidino-benzyl)-aminocarbonylmethyl]-benzoesäure

Hergestellt aus 2-Äthoxy-4-[N-(α-phenyl-2-piperidino-benzyl)-aminocarbonylmethyl]-benzoesäure-äthylester.
Ausbeute: 89% der Theorie,
Schmelzpunkt: 155–156 °C
Ber.: C 73,70 H 6,83 N 5,93
Gef.: C 73,60 H 6,96 N 6,12

c) 2-Methoxy-4-[N-(α-phenyl-2-piperidino-benzyl)-aminocarbonylmethyl]-benzoesäure

Hergestellt aus 2-Methoxy-4-[N-(α-phenyl-2-piperidino-benzyl)-aminocarbonylmethyl]-benzoesäure-methylester.
Ausbeute: 68% der Theorie,
Schmelzpunkt: 202–204 °C
Ber.: C 73,34 H 6,59 N 6,11
Gef.: C 73,60 H 6,77 N 6,20

d) 2-Äthoxy-4-[N-(1-(5-chlor-2-piperidino-phenyl)-1-propyl)-aminocarbonylmethyl]-benzoesäure

Hergestellt aus 2-Äthoxy-4-[N-(1-(5-chlor-2-piperidino-phenyl)-1-propyl)-aminocarbonylmethyl]-benzoesäure-äthylester.
.Ausbeute: 74% der Theorie,
Schmelzpunkt: 115–118 °C
Ber.: C 65,42 H 6,81 N 6,10 Cl 7,72
Gef.: C 65,54 H 6,94 N 5,81 Cl 7,89

e) 2-Äthoxy-4-[N-(1-(2-piperidino-phenyl)-1-propyl)-aminocarbonylmethyl]-benzoesäure

Hergestellt aus 2-Äthoxy-4-[N-(1-(2-piperidino-phenyl)-1-propyl)-aminocarbonylmethyl]-benzoesäure-äthylester.
Ausbeute: 73% der Theorie,
Schmelzpunkt: 81–83 °C
Ber.: C 70,73 H 7,60 N 6,60
Gef.: C 70,90 H 7,47 N 6,77

f) 2-Äthoxy-4-[N-(1-(2-piperidino-phenyl)-1-pentyl)-aminocarbonylmethyl]-benzoesäure

Hergestellt aus 2-Äthoxy-4-[N-(1-(2-piperidino-phenyl)-1-pentyl)-aminocarbonylmethyl]-benzoesäure-äthylester.
Ausbeute: 92% der Theorie,
Schmelzpunkt: 82–85 °C
Ber.: C 71,65 H 8,02 N 6,19
Gef.: C 71,45 H 8,01 N 6,13

g) 2-Äthoxy-4-[N-(1-(2-pyrrolidino-phenyl)-1-butyl)-aminocarbonylmethyl]-benzoesäure

Hergestellt aus 2-Äthoxy-4-[N-(1-(2-pyrrolidino-phenyl)-1-butyl)-aminocarbonylmethyl]-benzoesäure-äthylester.
Ausbeute: 77% der Theorie,
Schmelzpunkt: 120–123 °C
Ber.: C 70,33 H 7,60 N 6,60
Gef.: C 70,71 H 7,44 N 6,33

h) 2-Äthoxy-4-[N-(1-(2-(4-methyl-piperidino)-phenyl)-1-butyl)-aminocarbonylmethyl]-benzoesäure

Hergestellt aus 2-Äthoxy-4-[N-(1-(2-(4-methyl-piperidino)-phenyl)-1-butyl)-aminocarbonylmethyl]-benzoesäure-äthylester.
Ausbeute: 71% der Theorie,
Schmelzpunkt: 83–85 °C
Ber.: C 71,65 H 8,02 N 6,19
Gef.: C 71,60 H 7,94 N 6,09

i) 2-Äthoxy-4-[N-(1-(2-hexamethylenimino-phenyl)-1-butyl)-aminocarbonylmethyl]-benzoesäure

Hergestellt aus 2-Äthoxy-4-[N-(1-(2-hexamethylenimino-phenyl)-1-butyl)-aminocarbonylmethyl]-benzoesäure-äthylester.
Ausbeute: 81% der Theorie,
Schmelzpunkt: 101–105 °C
Ber.: C 71,65 H 8,02 N 6,19
Gef.: C 71,31 H 7,79 N 6,18

k) 2-Äthoxy-4-[N-(1-(6-chlor-2-piperidino-phenyl)-1-butyl)-aminocarbonylmethyl]-benzoesäure

Hergestellt aus 2-Äthoxy-4-[N-(1-(6-chlor-2-piperidino-phenyl)-1-butyl)-aminocarbonylmethyl]-benzoesäure-äthylester.
Ausbeute: 82% der Theorie,
Schmelzpunkt: 133–136 °C
Ber.: C 66,02 H 7,03 N 5,92 Cl 7,50
Gef.: C 66,48 H 7,47 N 5,98 Cl 7,88

l) 2-Äthoxy-4-[N-(1-(4-methoxy-2-piperidino-phenyl)-1-butyl)-aminocarbonylmethyl]-benzoesäure

Hergestellt aus 2-Äthoxy-4-[N-(1-(4-methoxy-2-piperidino-phenyl)-1-butyl)-aminocarbonylmethyl]-benzoesäure-äthylester.
Ausbeute: 81% der Theorie,
Schmelzpunkt: 98–100 °C
Ber.: C 69,21 H 7,74 N 5,98
Gef.: C 69,12 H 7,62 N 5,78

m) 2-Äthoxy-4-[N-(1-(5-methoxy-2-piperidino-phenyl)-1-butyl)-aminocarbonylmethyl]-benzoesäure

Hergestellt aus 2-Äthoxy-4-[N-(1-(5-methoxy-2-piperidino-phenyl)-1-butyl)-aminocarbonyl-methyl]-benzoesäure-äthylester.
Ausbeute: 74% der Theorie,
Schmelzpunkt: 145–148 °C

| | | | |
|---|---|---|---|
| Ber.: | C 69,21 | H 7,74 | N 5,98 |
| Gef.: | C 69,00 | H 7,65 | N 5,89 |

n) 2-Methoxy-4-[N-(1-(2-piperidino-phenyl)-1-butyl)-aminocarbonylmethyl]-benzoesäure

Hergestellt aus 2-Methoxy-4-[N-(1-(2-piperidino-phenyl)-1-butyl)-aminocarbonylmethyl]-benzoesäure-methylester.
Ausbeute: 86% der Theorie,
Schmelzpunkt: 140–143 °C

| | | | |
|---|---|---|---|
| Ber.: | C 70,73 | H 7,60 | N 6,60 |
| Gef.: | C 70,49 | H 7,58 | N 6,31 |

o) 2-Äthoxy-4-[N-(5-chlor-2-piperidino-benzyl)-aminocarbonylmethyl]-benzoesäure x 0,5 $H_2O$

Hergestellt aus 2-Äthoxy-4-[N-(5-chlor-2-piperidino-benzyl)-aminocarbonylmethyl]-benzoesäure-äthylester.
Ausbeute: 93% der Theorie,
Schmelzpunkt: 153–155 °C

| | | | | |
|---|---|---|---|---|
| Ber.: | C 62,79 | H 6,41 | N 6,36 | Cl 8,06 |
| Gef.: | C 63,21 | H 6,34 | N 5,89 | Cl 8,46 |

p) 2-Äthoxy-4-[N-(2-piperidino-benzyl)-aminocarbonylmethyl]-benzoesäure

Hergestellt aus 2-Äthoxy-4-[N-(2-piperidino-benzyl)-aminocarbonylmethyl]-benzoesäure-äthylester.
Ausbeute: 77% der Theorie,
Schmelzpunkt: 108–109 °C

| | | | |
|---|---|---|---|
| Ber.: | C 69,68 | H 7,12 | N 7,07 |
| Gef.: | C 70,00 | H 7,99 | N 7,31 |

q) 2-Hydroxy-4-[N-(1-(2-piperidino-phenyl)-1-butyl)-aminocarbonylmethyl]-benzoesäure

Hergestellt aus 2-Hydroxy-4-[N-(1-(2-piperidino-phenyl)-1-butyl)-aminocarbonylmethyl]-benzoesäure-äthylester.
Ausbeute: 61% der Theorie,
Schmelzpunkt: 136–138 °C

| | | | |
|---|---|---|---|
| Ber.: | C 70,22 | H 7,37 | N 6,82 |
| Gef.: | C 70,40 | H 7,64 | N 6,60 |

r) 2-Allyloxy-4-[N-(1-(2-piperidino-phenyl)-1-butyl)-aminocarbonylmethyl]-benzoesäure

Hergestellt aus 2-Allyloxy-4-[N-(1-(2-piperidino-phenyl)-1-butyl)-aminocarbonylmethyl]-benzoesäure-äthylester.
Ausbeute: 92% der Theorie,
Schmelzpunkt: 110–112 °C

| | | | |
|---|---|---|---|
| Ber.: | C 71,97 | H 7,61 | N 6,22 |
| Gef.: | C 71,90 | H 7,62 | N 6,21 |

s) (+)-2-Äthoxy-4-[N-(1-(2-piperidino-phenyl)-1-butyl)-aminocarbonylmethyl]-benzoesäure

Hergestellt aus (+)-2-Äthoxy-4-[N-(1-(2-piperidino-phenyl)-1-butyl)-aminocarbonylmethyl]-benzoesäure-äthylester.
Ausbeute: 81% der Theorie,
Schmelzpunkt: 122–123 °C

| | | | |
|---|---|---|---|
| Ber.: | C 71,21 | H 7,81 | N 6,39 |
| Gef.: | C 71,19 | H 7,77 | N 6,29 |

Spezifische Drehung $[\alpha]_D^{20}$ = +4,75° (c = 1,03 in Methanol)

t) 2-Äthoxy-4-[N-(4-methyl-α-phenyl-2-piperidino-benzyl)-aminocarbonylmethyl]-benzoesäure

Hergestellt aus 2-Äthoxy-4-[N-(4-methyl-α-phenyl-2-piperidino-benzyl)-aminocarbonylmethyl]-benzoesäure-äthylester.
Ausbeute: 41% der Theorie,
Schmelzpunkt: 127–129 °C

| | | | |
|---|---|---|---|
| Ber.: | C 74,05 | H 7,04 | N 5,76 |
| Gef.: | C 73,80 | H 7,09 | N 5,74 |

u) 2-Äthoxy-4-[N-(6-methyl-α-phenyl-2-piperidino-benzyl)-aminocarbonylmethyl]-benzoesäure

Hergestellt aus 2-Äthoxy-4-[N-(6-methyl-α-phenyl-2-piperidino-benzyl)-aminocarbonylmethyl]-benzoesäure-äthylester.
Ausbeute: 40% der Theorie,
Schmelzpunkt: 118–121 °C

| | | | |
|---|---|---|---|
| Ber.: | C 74,05 | H 7,04 | N 5,76 |
| Gef.: | C 73,71 | H 6,92 | N 5,76 |

v) 2-Äthoxy-4-[N-(α-(4-methyl-phenyl)-2-piperidino-benzyl)-aminocarbonylmethyl]-benzoesäure

Hergestellt aus 2-Äthoxy-4-[N-(α-(4-methyl-phenyl)-2-piperidino-benzyl)-aminocarbonylmethyl]-benzoesäure-äthylester.
Ausbeute: 94% der Theorie,
Schmelzpunkt: 148–151 °C

| | | | |
|---|---|---|---|
| Ber.: | C 74,05 | H 7,04 | N 5,76 |
| Gef.: | C 74,20 | H 7,15 | N 5,81 |

w) 2-Methoxy-4-[N-(α-(4-chlor-phenyl)-2-piperidino-benzyl)-aminocarbonylmethyl]-benzoesäure

Hergestellt aus 2-Methoxy-4-[N-(α-(4-chlor-phenyl)-2-piperidino-benzyl)-aminocarbonylmethyl]-benzoesäure-methylester.
Ausbeute: 77% der Theorie,
Schmelzpunkt: 177–180 °C

| | | | | |
|---|---|---|---|---|
| Ber.: | C 68,21 | H 5,93 | N 5,68 | Cl 7,19 |
| Gef.: | C 68,10 | H 5,78 | N 5,53 | Cl 7,43 |

x) 2-Äthoxy-4-[N-(α-phenyl-2-pyrrolidino-benzyl)-aminocarbonylmethyl]-benzoesäure

Hergestellt aus 2-Äthoxy-4-[N-(α-phenyl-2-pyrrolidino-benzyl)-aminocarbonylmethyl]-benzoesäure-äthylester.
Ausbeute: 67% der Theorie,
Schmelzpunkt: 141–143 °C

| | | | |
|---|---|---|---|
| Ber.: | C 73,34 | H 6,59 | N 6,11 |
| Gef.: | C 73,33 | H 6,74 | N 6,02 |

y) 2-Methoxy-4-N-(2-hexamethylenimino-α-phenyl-benzyl)-aminocarbonylmethyl]-benzoesäure

Hergestellt aus 2-Methoxy-4-N-(2-hexamethylenimino-α-phenyl-benzyl)-aminocarbonylmethyl]-benzoesäure-methylester.

Ausbeute: 90% der Theorie,
Schmelzpunkt: 154–156 °C

| | | | |
|---|---|---|---|
| Ber.: | C 73,70 | H 6,83 | N 5,93 |
| Gef.: | C 73,70 | H 7,00 | N 5,95 |

z) 2-Äthoxy-4-[N-(2-hexamethylenimino-α-phenyl-benzyl)-aminocarbonylmethyl]-benzoesäure

Hergestellt aus 2-Äthoxy-4-[N-(2-hexamethylenimino-α-phenyl-benzyl)-aminocarbonylmethyl]-benzoesäure-äthylester.

Ausbeute: 75% der Theorie,
Schmelzpunkt: 139–141 °C

| | | | |
|---|---|---|---|
| Ber.: | C 74,05 | H 7,04 | N 5,76 |
| Gef.: | C 73,90 | H 7,14 | N 5,79 |

aa) 2-Hydroxy-4-[N-(α-phenyl-2-piperidino-benzyl)-aminocarbonylmethyl]-benzoesäure

Hergestellt aus 2-Hydroxy-4-[N-(α-phenyl-2-piperidino-benzyl)-aminocarbonylmethyl]-benzoesäure-äthylester durch Verseifung mittels 4 Äquivalenten 1N-Natronlauge in Äthanol/Dioxan.

Ausbeute: 35% der Theorie,
Schmelzpunkt: 222–224 °C

| | | | |
|---|---|---|---|
| Ber.: | C 72,95 | H 6,35 | N 6,30 |
| Gef.: | C 73,00 | H 6,64 | N 6,28 |

ab) 2-Allyloxy-4-[N-(α-phenyl-2-piperidino-benzyl)-aminocarbonylmethyl]-benzoesäure

Hergestellt aus 2-Allyloxy-4-[N-(α-phenyl-2-piperidino-benzyl)-aminocarbonylmethyl]-benzoesäure-äthylester.

Ausbeute: 69% der Theorie,
Schmelzpunkt: 172–173 °C

| | | | |
|---|---|---|---|
| Ber.: | C 74,35 | H 6,66 | N 5,78 |
| Gef.: | C 74,11 | H 6,50 | N 5,74 |

ac) (−)-2-Äthoxy-4-[N-(α-phenyl-2-piperidino-benzyl)-aminocarbonylmethyl]-benzoesäure

Hergestellt aus (−)-2-Äthoxy-4-[N-(α-phenyl-2-piperidino-benzyl)-aminocarbonylmethyl]-benzoesäure-äthylester.

Ausbeute: 89% der Theorie,
Schmelzpunkt: 90–95 °C

| | | | |
|---|---|---|---|
| Ber.: | C 73,70 | H 6,83 | N 5,93 |
| Gef.: | C 73,59 | H 6,81 | N 5,83 |

Spezifische Drehung: $[\alpha]_D^{20}$ = −2,2° (c = 1 in Methanol)

Beispiel 5

2-Äthoxy-4-[N-(1-(2-piperidino-phenyl)-1-äthyl)-aminocarbonylmethyl]-benzoesäure-Natriumsalz x 1,5 $H_2O$

Hergestellt aus 2-Äthoxy-4-[N-(1-(2-piperidino-phenyl)-1-äthyl)-aminocarbonylmethyl]-benzoesäure-äthylester analog Beispiel 4. Nach der säulenchromatographischen Reinigung wird der erhaltene Eindampfrückstand in Äthanol gelöst und mit 1 Äquivalent 1N-Natronlauge versetzt. Durch Eindampfen im Vakuum und Verreiben mit Aceton erhält man das kristalline Natriumsalz.

Ausbeute: 76% der Theorie,
Schmelzpunkt: 242–244 °C

| | | | |
|---|---|---|---|
| Ber.: | C 62,73 | H 7,01 | N 6,01 |
| Gef.: | C 62,74 | H 7,17 | N 6,05 |

Analog Beispiel 5 wurden folgende Verbindungen erhalten:

a) 2-Äthoxy-4-[N-(1-(4-methyl-2-piperidino-phenyl)-1-butyl)-aminocarbonylmethyl]-benzoesäure-Natriumsalz x 0,5 $H_2O$

Hergestellt aus 2-Äthoxy-4-[N-(1-(4-methyl-2-piperidino-phenyl)-1-butyl)-aminocarbonylmethyl]-benzoesäure-äthylester.

Ausbeute: 72% der Theorie,
Schmelzpunkt: 255–260 °C

| | | | |
|---|---|---|---|
| Ber.: | C 67,06 | H 7,50 | N 5,79 |
| Gef.: | C 66,94 | H 7,28 | N 5,50 |

b) 2-Äthoxy-4-[N-(1-(6-methyl-2-piperidino-phenyl)-1-butyl)-aminocarbonylmethyl]-benzoesäure-Natriumsalz x 2,5 $H_2O$

Hergestellt aus 2-Äthoxy-4-[N-(1-(6-methyl-2-piperidino-phenyl)-1-butyl)-aminocarbonylmethyl]-benzoesäure-äthylester.

Ausbeute: 81% der Theorie,
Schmelzpunkt: 232–240 °C

| | | | |
|---|---|---|---|
| Ber.: | C 62,39 | H 7,75 | N 5,39 |
| Gef.: | C 62,22 | H 7,46 | N 5,61 |

c) 2-Äthoxy-4-[N-(1-(2-piperidino-phenyl)-1-butyl)-aminocarbonylmethyl]-benzoesäure-Natriumsalz

Hergestellt aus 2-Äthoxy-4-[N-(1-(2-piperidino-phenyl)-1-butyl)-aminocarbonylmethyl]-benzoesäure-äthylester.

Ausbeute: 87% der Theorie,
Schmelzpunkt: 250–258 °C

| | | | |
|---|---|---|---|
| Ber.: | C 67,79 | H 7,22 | N 6,08 |
| Gef.: | C 67,60 | H 7,37 | N 6,04 |

d) 2-Äthoxy-4-[N-(α-phenyl-2-piperidino-benzyl)-aminocarbonylmethyl]-benzoesäure-Natriumsalz

Hergestellt aus 2-Äthoxy-4-[N-(α-phenyl-2-piperidino-benzyl)-aminocarbonylmethyl]-benzoesäure-äthylester.

Ausbeute: 89% der Theorie,
Schmelzpunkt: 233–235 °C

| | | | |
|---|---|---|---|
| Ber.: | C 70,42 | H 6,32 | N 5,67 |
| Gef.: | C 70,20 | H 6,41 | N 5,49 |

Beispiel 6

2-Hydroxy-4-[N-(α-phenyl-2-piperidino-benzyl)-aminocarbonylmethyl]-benzoesäure-äthylester

Zu einer gerührten Lösung von 2 g (4 mMol) 2-Äthoxy-4-[N-(α-phenyl-2-piperidino-benzyl)-aminocarbonylmethyl]-benzoesäure-äthylester in 20 ml 1,2-Dichloräthan gibt man unter Feuchtigkeitsausschluß bei −20 °C tropfenweise 1 ml (10,4 mMol) Bortribromid. Man läßt auf Raumtemperatur kommen und rührt noch 17 Stunden. Dann gießt man in Äthanol ein, engt im Vakuum ein, setzt Eis zu und verteilt zwischen Chloroform und Wasser. Den organischen Extrakt trocknet und filtriert man und dampft ihn im Vakuum ein. Der Eindampfrückstand wird säulenchromatographisch an Kieselgel (Toluol/Äthylacetat = 5/1) gereinigt.
Ausbeute: 0,37 g (21% der Theorie),
Schmelzpunkt: 172–173 °C

| | | | |
|---|---|---|---|
| Ber.: | C 72,70 | H 6,83 | N 5,93 |
| Gef.: | C 73,95 | H 7,05 | N 6,12 |

Analog Beispiel 6 wurden folgende Verbindungen erhalten:

a) 2-Hydroxy-4-[N-(α-phenyl-2-piperidino-benzyl)-aminocarbonylmethyl]-benzoesäure

Hergestellt aus 2-Äthoxy-4-[N-(α-phenyl-2-piperidino-benzyl)-aminocarbonylmethyl]-benzoesäure.
Ausbeute: 40% der Theorie,
Schmelzpunkt: 221–223 °C

| | | | |
|---|---|---|---|
| Ber.: | C 72,95 | H 6,35 | N 6,30 |
| Gef.: | C 72,68 | H 6,45 | N 6,49 |

b) 2-Hydroxy-4-[N-(1-(2-piperidino-phenyl)-1-butyl)-aminocarbonylmethyl]-benzoesäure-äthylester

Hergestellt aus 2-Äthoxy-4-[N-(1-(2-piperidino-phenyl)-1-butyl)-aminocarbonylmethyl]-benzoesäure-äthylester.
Ausbeute: 19% der Theorie,
Schmelzpunkt: 132–134 °C

| | | | |
|---|---|---|---|
| Ber.: | C 71,21 | H 7,81 | N 6,39 |
| Gef.: | C 71,43 | H 7,91 | N 6,55 |

c) 2-Hydroxy-4-[N-(1-(2-piperidino-phenyl)-1-butyl)-aminocarbonylmethyl]-benzoesäure

Hergestellt aus 2-Äthoxy-4-[N-(1-(2-piperidino-phenyl)-1-butyl)-aminocarbonylmethyl]-benzoesäure.
Ausbeute: 42% der Theorie,
Schmelzpunkt: 136–137 °C

| | | | |
|---|---|---|---|
| Ber.: | C 70,22 | H 7,37 | N 6,82 |
| Gef.: | C 70,19 | H 7,39 | N 6,99 |

Beispiel 7

2-Äthoxy-4-[N-(1-(2-piperidino-phenyl)-1-butyl)-aminocarbonylmethyl]-benzoesäure-tert.butylester

Man rührt das Gemisch aus 1,98 g (9,6 mMol) N,N-Dicyclohexylcarbodiimid, 1,06 ml (11,2 mMol) absolutes tert.Butanol und 0,020 g (0,20 mMol) Kupfer(I)chlorid 60 Stunden bei Raumtemperatur, gibt dann 6,6 ml Methylenchlorid zu und tropft die so erhaltene Lösung zur Lösung von 0,44 g (1 mMol) 2-Äthoxy-4-[N-(1-(2-piperidino-phenyl)-1-butyl)-aminocarbonylmethyl]-benzoesäure in 15 ml Methylenchlorid. Nach 60 Stunden Rühren bei 20 °C filtriert man vom ausgefallenen Niederschlag ab, wäscht ihn mit Methylenchlorid und dampft die Methylenchlorid-Lösung im Vakuum ein. Den Eindampfrückstand reinigt man durch Säulenchromatographie an Kieselgel (Chloroform/Äthylacetat = 9/1).
Ausbeute: 0,30 g (60% der Theorie),
Schmelzpunkt: 74–77 °C (aus Petroläther)

| | | | |
|---|---|---|---|
| Ber.: | C 72,84 | H 8,56 | N 5,66 |
| Gef.: | C 73,00 | H 8,65 | N 5,79 |

Beispiel 8

2-Allyloxy-4-[N-(α-phenyl-2-piperidino-benzyl)-aminocarbonylmethyl]-benzoesäure-äthylester

Zu einer Lösung von 1,1 g (2,3 mMol) 2-Hydroxy-4-[N-(α-phenyl-2-piperidino-benzyl)-aminocarbonylmethyl]-benzoesäure-äthylester in 10 ml wasserfreiem Dimethylformamid gibt man 0,10 g (2,3 mMol) Natriumhydrid (55%ig in Öl) und rührt 0,5 Stunden bei Raumtemperatur. Dann tropft man die Lösung von 0,27 ml (2,3 mMol) Allylbromid in 5 ml wasserfreiem Dimethylformamid zu und rührt 5 Stunden bei Raumtemperatur. Man dampft im Vakuum ein, verteilt zwischen verdünnter Natronlauge und Chloroform, trocknet den organischen Extrakt, filtriert ihn und dampft ihn im Vakuum ein. Den Eindampfrückstand kristallisiert man aus Acetonitril und anschließend aus Äthanol um.
Ausbeute: 0,55 g (46% der Theorie),
Schmelzpunkt: 117–119 °C

| | | | |
|---|---|---|---|
| Ber.: | C 74,97 | H 7,08 | N 5,47 |
| Gef.: | C 74,90 | H 7,14 | N 5,38 |

Analog Beispiel 8 wurde folgende Verbindung erhalten:

a) 2-Allyloxy-4-[N-(1-(2-piperidino-phenyl)-1-butyl)-aminocarbonylmethyl]-benzoesäure-äthylester

Hergestellt aus 2-Hydroxy-4-[N-(1-(2-piperidino-phenyl)-1-butyl)-aminocarbonylmethyl]-benzoesäure-äthylester mittels Allylbromid.
Ausbeute: 72% der Theorie,
Schmelzpunkt: 105–106 °C

| | | | |
|---|---|---|---|
| Ber.: | C 72,77 | H 8,00 | N 5,85 |
| Gef.: | C 72,90 | H 7,90 | N 5,87 |

Beispiel 9

2-Äthoxy-4-[N-(2-piperidino-benzyl)-aminocarbonylmethyl]-benzoesäure-äthylester

1,0 g (2,18 mMol) 2-Äthoxy-4-[N-(5-chlor-2-piperidino-benzyl)-aminocarbonylmethyl]-benzoesäure-äthylester wird in 20 ml Äthanol an 0,5 g Palladium/Kohle (10%ig) bei 50 °C und 1 bar Wasserstoff 45 Minuten lang hydriert. Man filtriert über Kieselgur, dampft im Vakuum ein und reinigt durch Säulenchromatographie an Kieselgel (Chloroform/Methanol = 10/1).
Ausbeute: 0,71 g (77% der Theorie),
Schmelzpunkt: 83–84 °C (aus Petroläther)

| | | | |
|---|---|---|---|
| Ber.: | C 70,73 | H 7,60 | N 6,60 |
| Gef.: | C 70,89 | H 7,66 | N 6,76 |

Analog Beispiel 9 wurde folgende Verbindung erhalten:

a) 2-Äthoxy-4-[N-(1-(2-piperidino-phenyl)-1-propyl)-aminocarbonylmethyl]-benzoesäure-äthylester

Hergestellt aus 2-Äthoxy-4-[N-(1-(5-chlor-2-piperidino-phenyl)-1-propyl)-aminocarbonylmethyl]-benzoesäure-äthylester.
Ausbeute: 74% der Theorie,
Schmelzpunkt: 115–117 °C
Ber.: C 71,65 H 8,02 N 6,19
Gef.: C 71,47 H 8,11 N 6,25

Beispiel 10
2-Äthoxy-4-[N-(1-(2-piperidino-phenyl)-3-methyl-1-butyl)-aminocarbonylmethyl]-benzoesäure

(i) 2-Äthoxy-4-[N-(1-(2-piperidino-phenyl)-3-methyl-1-buten-1-yl)-aminocarbonylmethyl]-benzoesäure

Hergestellt aus 2-Äthoxy-4-[N-(1-(2-piperidino-phenyl)-3-methyl-1-buten-1-yl)-aminocarbonylmethyl]-benzoesäure-äthylester.
Ausbeute: 85% der Theorie,
Schmelzpunkt: 110–113 °C
Ber.: C 71,97 H 7,61 N 6,22
Gef.: C 71,92 H 7,80 N 5,98

(ii) 0,21 g (0,39 mMol) 2-Äthoxy-4-[N-(1-(2-piperidino-phenyl)-3-methyl-1-buten-1-yl)-aminocarbonylmethyl]-benzoesäure hydriert man in 10 ml absolutem Äthanol an 0,10 g Palladium/Kohle (10%ig) bei 50 °C und 1 bar Wasserstoff 7 Stunden lang. Man filtriert über Kieselgur, dampft im Vakuum ein und reinigt durch Säulenchromatographie an Kieselgel (Chloroform/Methanol = 10/1).
Ausbeute: 0,10 g (47% der Theorie),
Schmelzpunkt: 90–92 °C
Ber.: C 71,65 H 8,02 N 6,19
Gef.: C 71,50 H 8,12 N 6,45

Analog Beispiel 10 wurde folgende Verbindung erhalten:
a) 2-Äthoxy-4-[N-(1-(2-piperidino-phenyl)-3-methyl-1-butyl)-aminocarbonylmethyl]-benzoesäure-äthylester

Hergestellt aus 2-Äthoxy-4-[N-(1-(2-piperidino-phenyl)-3-methyl-1-buten-1-yl)-aminocarbonylmethyl]-benzoesäure-äthylester vom Schmelzpunkt 125–126 °C [hergestellt aus (2-Piperidino-phenyl)-isobutyl-ketimin und 3-Äthoxy-4-äthoxycarbonyl-phenylessigsäure analog Beispiel 1].
Ausbeute: 51% der Theorie,
Schmelzpunkt: 139–141 °C
Ber.: C 72,47 H 8,39 N 5,83
Gef.: C 72,30 H 8,20 N 5,87

Beispiel 11
2-Äthoxy-4-[N-(α-phenyl-2-piperidino-benzyl)-aminocarbonylmethyl]-benzylalkohol

Zu 0,28 g (7,4 mMol) Lithiumaluminiumhydrid in 50 ml absolutem Tetrahydrofuran tropft man bei −5 °C die Lösung von 1,8 g (3,6 mMol) 2-Äthoxy-4-[N-(α-phenyl-2-piperidino-benzyl)-aminocarbonylmethyl]-benzoesäure-äthylester in 20 ml absolutem Tetrahydrofuran und rührt 3 Stunden bei 0 °C. Dann verdünnt man mit absolutem Äther und versetzt mit 4N-Natronlauge. Man filtriert über Kieselgur, dampft im Vakuum ein und reinigt durch Säulenchromatographie an Kieselgel (Toluol/Äthylacetat = 2/1).
Ausbeute: 0,51 g (31% der Theorie),
Schmelzpunkt: 133–135 °C
Ber.: C 75,95 H 7,47 N 6,11
Gef.: C 75,97 H 7,55 N 5,95

Analog Beispiel 11 wurde folgende Verbindung erhalten:
a) 2-Äthoxy-4-[N-(1-(2-piperidino-phenyl)-1-butyl)-aminocarbonylmethyl]-benzylalkohol

Hergestellt aus 2-Äthoxy-4-[N-(1-(2-piperidino-phenyl)-1-butyl)-aminocarbonylmethyl]-benzoesäure-äthylester durch Reduktion mit Lithiumborhydrid in siedendem Tetrahydrofuran unter Zusatz von 10% Borsäure-trimethylester.
Ausbeute: 68% der Theorie,
Schmelzpunkt: 112–115 °C
Ber.: C 73,55 H 8,55 N 6,60
Gef.: C 73,60 H 8,38 N 6,69

Beispiel 12
2-Äthoxy-4-[N-(α-phenyl-2-piperidino-benzyl)-aminocarbonylmethyl]-benzaldehyd

Zur gerührten Lösung von 0,28 g (1,3 mMol) Pyridiniumchlorochromat in 5 ml Chloroform tropft man bei Raumtemperatur die Lösung von 0,4 g (0,87 mMol) 2-Äthoxy-4-[N-(α-phenyl-2-piperidino-benzyl)-aminocarbonylmethyl]-benzylalkohol. Man rührt über Nacht bei Raumtemperatur, dampft im Vakuum ein, versetzt mit Äther, filtriert, dampft im Vakuum ein und reinigt durch Säulenchromatographie an Kieselgel (Toluol/Äthylacetat = 2/1).
Ausbeute: 0,16 g (40% der Theorie),
Schmelzpunkt: 154–156 °C
Ber.: C 76,29 H 7,06 N 6,14
Gef.: C 76,30 H 7,15 N 6,10

Analog Beispiel 12 wurde folgende Verbindung erhalten:
a) 2-Äthoxy-4-[N-(1-(2-piperidino-phenyl)-1-butyl)-aminocarbonylmethyl]-benzaldehyd

Hergestellt aus 2-Äthoxy-4-[N-(1-(2-piperidino-phenyl)-1-butyl)-aminocarbonylmethyl]-benzylalkohol.
Ausbeute: 47% der Theorie,
Schmelzpunkt: 109–111 °C
Ber.: C 73,90 H 8,11 N 6,63
Gef.: C 74,22 H 8,14 N 6,73

Beispiel 13
2-Äthoxy-4-[N-(α-phenyl-2-piperidino-benzyl)-aminocarbonylmethyl]-benzaldehyd

Man erhitzt 0,67 g (5,6 mMol) Natriumcarbonat zusammen mit 6 ml Äthylenglykol im Bad von 170 °C und gibt unter raschem Rühren innerhalb einer Minute 0,70 g (1,1 mMol) $N^1$-[2-Äthoxy-4-[N-(α-phenyl-2-piperidino-benzyl)-aminocarbonylmethyl]-benzoyl]-$N^2$-tosyl-hydrazin zu, wobei man eine heftige Gasentwicklung beobachtet. Anschließend erhitzt man noch 2 Minuten bei 170 °C und gießt dann sofort auf Eis. Man extrahiert mit Äther, trocknet, filtriert und dampft die Ätherlösung im Vakuum ein. Den Eindampfrückstand reinigt man durch Säulenchromatographie an Kieselgel (Toluol/Äthylacetat = 2/1).
Ausbeute: 0,25 g (50% der Theorie),
Schmelzpunkt: 153–156 °C

| | | | |
|---|---|---|---|
| Ber.: | C 76,29 | H 7,06 | N 6,14 |
| Gef.: | C 76,42 | H 7,33 | N 6,28 |

Analog Beispiel 13 wurde folgende Verbindung erhalten:
a) 2-Äthoxy-4-[N-(1-(2-piperidino-phenyl)-1-butyl)-aminocarbonylmethyl]-benzaldehyd

Hergestellt aus $N^1$-[2-Äthoxy-4-[N-(1-(2-piperidino-phenyl)-1-butyl)-aminocarbonylmethyl]-benzoyl]-$N^2$-tosyl-hydrazin.
Ausbeute: 51% der Theorie,
Schmelzpunkt: 108–111 °C

| | | | |
|---|---|---|---|
| Ber.: | C 73,90 | H 8,11 | N 6,63 |
| Gef.: | C 73,79 | H 8,29 | N 6,75 |

Beispiel 14

2-Äthoxy-4-[N-(1-(2-piperidino-phenyl)-1-butyl)-aminocarbonylmethyl]-benzoesäure-benzylester

Man erhitzt 0,35 g (0,8 mMol) 2-Äthoxy-4-[N-(1-(2-piperidino-phenyl)-1-butyl)-aminocarbonylmethyl]-benzoesäure zusammen mit 0,15 g (0,9 mMol) N,N'-Carbonyldiimidazol in 15 ml absolutem Tetrahydrofuran 2 Stunden am Rückfluß. Dann gibt man 1,03 ml (10 mMol) Benzylalkohol zu und erhitzt 3,5 Stunden am Rückfluß. Man dampft im Vakuum ein und reinigt durch Säulenchromatographie an Kieselgel (Chloroform/Aceton = 9/1).
Ausbeute: 0,10 g (23,6% der Theorie),
Schmelzpunkt: <20 °C

| | |
|---|---|
| Ber.: | Molpeak m/e = 528 |
| Gef.: | Molpeak m/e = 528 |

Beispiel 15

(+)-2-Äthoxy-4-[N-(1-(2-piperidino-phenyl)-1-butyl)-aminocarbonylmethyl]-benzoesäure-äthylester und
(−)-2-Äthoxy-4-[N-(1-(2-piperidino-phenyl)-1-butyl)-aminocarbonylmethyl]-benzoesäure-äthylester

28 mg (±)-2-Äthoxy-4-[N-(1-(2-piperidino-phenyl)-1-butyl)-aminocarbonylmethyl]-benzoesäure-äthylester werden in Einzeldosen à 0,02 mg auf eine Chiral-Phasen-HPLC-Säule der Firma Baker aufgegeben, bei der (R)-N-3,5-Dinitrobenzoyl-phenylglycin kovalent an Aminopropyl-Silicagel gebunden ist (5 µm Korngröße, kugelförmig, 60A Porenweite; 4,6 mm Innendurchmesser, 25 cm Länge).
Fließmittel: Hexan/Äthanol = 100/5;
Fließgeschwindigkeit: 0,75 ml/Minute;
Temperatur: 22 °C.

Die nach 31,2 Minuten und nach 32,9 Minuten (UV-Detektion bei 254 nm) eluierten Fraktionen werden getrennt aufgefangen, gesammelt und im Vakuum eingedampft.

Aus dem 31,2 Minuten-Eluat erhält Iman:
7,5 mg (+)-2-Äthoxy-4-[N-(1-(2-piperidino-phenyl)-1-butyl)-aminocarbonylmethyl]-benzoesäure-äthylester
Schmelzpunkt: 117–119 °C
Spezifische Drehung: $[\alpha]_D^{20}$ = +7,0° (c = 1,03 in Methanol)

Aus dem 32,9 Minuten-Eluat erhält man:
9,4 mg (−)-2-Äthoxy-4-[N-(1-(2-piperidino-phenyl)-1-butyl)-aminocarbonylmethyl]-benzoesäure-äthylester
Schmelzpunkt: 115–117 °C
Spezifische Drehung: $[\alpha]_D^{20}$ = −6,9° (c = 1,02 in Methanol)

Analog Beispiel 15 läßt sich
a) (±)-2-Äthoxy-4-[N-(α-phenyl-2-piperidino-benzyl)-aminocarbonylmethyl]-benzoesäure-äthylester
in sein (+)-Enantiomeres und sein (−)-Enantiomeres auftrennen.

Beispiel 16

2-Äthoxy-4-[N-(1-(2-piperidino-phenyl)-1-butyl)-aminocarbonyl]-toluol

Man hydriert 0,54 g (1,2 mMol) 2-Äthoxy-4-[N-(1-(2-piperidino-phenyl)-1-butyl)-aminocarbonylmethyl]-benzylchlorid [Schmelzpunkt: 114–115 °C; hergestellt aus 2-Äthoxy-4-[N-(1-(2-piperidino-phenyl)-1-butyl)-aminocarbonylmethyl]-benzylalkohol mittels Thionylchlorid in Chloroform] in 10 ml absolutem Dioxan 3 Stunden bei 20 °C und 5 bar Wasserstoff. Man dampft im Vakuum ein und verteilt zwischen Äthylacetat und wäßriger Natriumcarbonatlösung. Den organischen Extrakt trocknet und filtriert man und dampft ihn im Vakuum ein. Den Eindampfrückstand reinigt man durch Säulenchromatographie an Kieselgel (Chloroform/Aceton = 19/1).
Ausbeute: 0,23 g (47% der Theorie),
Schmelzpunkt: 107–108 °C

| | | | |
|---|---|---|---|
| Ber.: | C 76,43 | H 8,88 | N 6,86 |
| Gef.: | C 76,40 | H 8,88 | N 6,90 |

Beispiel 17

2-Äthoxy-4-[N-(1-(2-piperidino-phenyl)-1-butyl)-aminocarbonylmethyl]-benzoesäure

Man erhitzt 100 mg (0,20 mMol) 2-Äthoxy-4-[N-(1-(2-piperidino-phenyl)-1-butyl)-aminocarbonyl]-benzoesäure-tert.butylester in 5 ml Benzol zusammen mit einigen Kristallen p-Toluolsulfonsäure-hydrat einen halben Tag auf Rückfluß. Dann ist laut Dünnschichtchromatogramm nach $R_f$-Wert und Massenspektrum das gewünschte Produkt entstanden.
Schmelzpunkt: 87–89 °C

| | |
|---|---|
| Ber.: | m/e = 438 |
| Gef.: | m/e = 438 |

Beispiel 18

2-Äthoxy-4-[N-(1-(2-piperidino-phenyl)-1-butyl-aminocarbonylmethyl]-benzoesäure

Man hydriert 0,25 g (0,47 mMol) 2-Äthoxy-4-[N-(1-(2-piperidino-phenyl)-1-butyl-aminocarbonyl-methyl]-benzoesäure-benzylester in 10 ml Äthanol an 0,12 g Palladium/Kohle (10%ig) bei 50 °C und 5 bar Wasserstoff. Nach 5 Stunden filtriert man vom Katalysator über Kieselgur ab und dampft im Vakuum ein. Den Eindampfrückstand kristallisiert man aus Petroläther/Äthanol.

Ausbeute: 0,14 g (70% der Theorie),
Schmelzpunkt: 87–90 °C

| | | | |
|---|---|---|---|
| Ber.: | C 71,21 | H 7,81 | N 6,39 |
| Gef.: | C 71,46 | H 7,95 | N 6,51 |

Beispiel 19

2-Äthoxy-4-[N-(1-(2-piperidino-phenyl)-1-n-hexyl)-aminocarbonylmethyl]-benzoesäure-äthylester

Hergestellt aus 1-(2-Piperidino-phenyl)-1-n-hexylamin und 3-Äthoxy-4-äthoxycarbonyl-phenylessigsäure analog Beispiel 1.

Ausbeute: 43% der Theorie,
Schmelzpunkt: 101–105 °C

| | | | |
|---|---|---|---|
| Ber.: | C 72,84 | H 8,56 | N 5,66 |
| Gef.: | C 72,72 | H 8,52 | N 5,63 |

Beispiel 20

2-Äthoxy-4-[N-(1-(2-piperidino-phenyl)-1-n-hexyl)-aminocarbonylmethyl]-benzoesäure

Hergestellt aus 2-Äthoxy-4-[N-(1-(2-piperidino-phenyl)-1-n-hexyl)-aminocarbonylmethyl]-benzoesäure-äthylester analog Beispiel 4.

Ausbeute: 77% der Theorie,
Schmelzpunkt: 117–120 °C

| | | | |
|---|---|---|---|
| Ber.: | C 72,07 | H 8,21 | N 6,00 |
| Gef.: | C 72,00 | H 8,06 | N 5,90 |

Beispiel A

Tabletten mit 5 mg 2-Äthoxy-4-[N-(α-phenyl-2-piperidino-benzyl)-aminocarbonylmethyl]-benzoesäure

Zusammensetzung:
1 Tablette enthält:

| | | |
|---|---|---|
| Wirksubstanz | (1) | 5,0 mg |
| Maisstärke | (2) | 62,0 mg |
| Milchzucker | (3) | 48,0 mg |
| Polyvinylpyrrolidon | (4) | 4,0 mg |
| Magnesiumstearat | (5) | 1,0 mg |
| | | 120,0 mg |

Herstellungsverfahren:

1, 2, 3 und 4 werden gemischt und mit Wasser befeuchtet. Die feuchte Mischung wird durch ein Sieb mit 1,5 mm Maschenweite gedrückt und bei ca. 45 °C getrocknet. Das trockene Granulat wird durch ein Sieb mit 1,0 mm Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg

Beispiel B

Dragées mit 2,5 mg 2-Äthoxy-4-[N-(α-phenyl-2-piperidino-benzyl)-aminocarbonylmethyl]-benzoesäure

1 Dragéekern enthält:

| | | |
|---|---|---|
| Wirksubstanz | (1) | 2,5 mg |
| Kartoffelstärke | (2) | 44,0 mg |
| Milchzucker | (3) | 30,0 mg |
| Polyvinylpyrrolidon | (4) | 3,0 mg |
| Magnesiumstearat | (5) | 0,5 mg |
| | | 80,0 mg |

Herstellungsverfahren:

1, 2, 3 und 4 werden gut gemischt und mit Wasser befeuchtet. Die feuchte Masse drückt man durch ein Sieb mit 1 mm Maschenweite, trocknet bei ca. 45 °C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 120 mg

Beispiel C

Tabletten mit 10 mg 2-Äthoxy-4-[N-(α-phenyl-2-piperidino-benzyl)-aminocarbonylmethyl]-benzoesäure

Zusammensetzung:
1 Tablette enthält:

| | |
|---|---|
| Wirksubstanz | 10,0 mg |
| Milchzucker pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren:

Die Mischung aus der Wirksubstanz, Milchzucker und Maisstärke wird mit einer 20%igen Lösung von Polyvinylpyrrolidon in Wasser befeuchtet. Die feuchte Masse wird durch ein Sieb mit 1,5 mm Maschenweite granuliert und bei 45 °C getrocknet. Das getrocknete Granulat wird durch ein Sieb mit 1 mm Maschenweite gerieben und mit Magnesiumstearat homogen vermischt.

Tablettengewicht: 120 mg
Stempel: 7 mm Durchmesser mit Teilkerbe

Beispiel D

Dragées mit 5 mg 2-Äthoxy-4-[N-(α-phenyl-2-piperidino-benzyl)-aminocarbonylmethyl]-benzoesäure

1 Dragéekern enthält:

| | |
|---|---|
| Wirksubstanz | 5,0 mg |
| Calciumphosphat sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren:

Die Mischung aus der Wirksubstanz, Calciumphosphat und Maisstärke wird mit einer 15%igen Lösung von Polyvinylpyrrolidon in Wasser befeuchtet. Die feuchte Masse wird durch ein Sieb mit 1 mm Maschenweite geschlagen, bei 45 °C getrocknet und anschließend durch dasselbe Sieb gerieben. Nach dem Vermischen mit der angegebenen Menge Magnesiumstearat werden daraus Dragéekerne gepreßt.

Kerngewicht: 130 mg
Stempel: 7 mm Durchmesser

Auf die so hergestellten Dragéekerne wird auf bekannte Art eine Schicht aus Zucker und Talkum aufgetragen. Die fertigen Dragées werden mit Wachs poliert.

Dragéegewicht: 180 mg

**Patentansprüche für die Vertragsstaaten: BE, CH, DE, FR, GB, IT, LI, LU, NL, SE**

1. Phenylessigsäurederivate der allgemeinen Formel

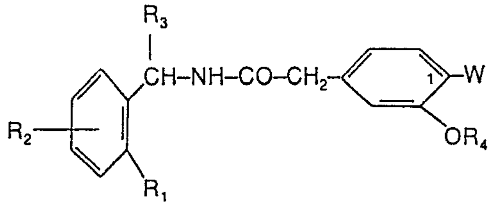

, (I)

in der

$R_1$ eine Pyrrolidino-, Piperidino-, Hexamethylenimino-, Methyl-pyrrolidino-, Dimethyl-pyrrolidino-, 2-Methyl-piperidino-, 3-Methyl-piperidino-, 4-Methylpiperidino-, 3,3-Dimethyl-piperidino-, cis-3,5-Dimethylpiperidino- oder trans-3,5-Dimethyl-piperidinogruppe,

$R_2$ ein Wasserstoff- oder Halogenatom, eine Methyl- oder Methoxygruppe,

$R_3$ ein Wasserstoffatom, eine Alkylgruppe mit 1 bis 4 Kohlenstoffatomen, eine n-Pentyl- oder 3-Methyl-n-butylgruppe oder eine gegebenenfalls durch ein Halogenatom, eine Methyl- oder Methoxygruppe substituierte Phenylgruppe,

$R_4$ ein Wasserstoffatom, eine Methyl-, Äthyl- oder Allylgruppe und

W eine Methyl-, Hydroxymethyl-, Formyl-, Carboxy- oder Alkoxycarbonylgruppe mit insgesamt 2 bis 5 Kohlenstoffatomen, wobei der Alkylteil der Alkoxygruppe durch eine Phenylgruppe substituiert sein kann, bedeuten, deren Enantiomere und deren Salze.

2. Phenylessigsäurederivate der allgemeinen Formel I, gemäß Anspruch 1, in der

$R_3$ und $R_4$ wie im Anspruch 1 definiert sind,

$R_1$ eine Pyrrolidino-, Piperidino-, 4-Methyl-piperidino-, 3-Methyl-piperidino-, 3,3-Dimethyl-piperidino-, 3,5-Dimethyl-piperidino- oder Hexamethyleniminogruppe,

$R_2$ ein Wasserstoff-, Fluor- oder Chloratom, eine Methyl- oder Methoxygruppe und

W eine Carboxy-, Methyl-, Hydroxymethyl-, Formyl- oder Benzyloxycarbonylgruppe oder eine Alkoxycarbonylgruppe mit insgesamt 2 bis 5 Kohlenstoffatomen darstellen, deren Enantiomere und deren Salze.

3. Phenylessigsäurederivate der allgemeinen Formel I gemäß Anspruch 1, in der

$R_1$ eine Piperidinogruppe,

$R_2$ ein Wasserstoff-, Fluor- oder Chloratom,

$R_3$ eine Methyl-, Äthyl-, n-Propyl-, n-Butyl-, Isobutyl-, n-Pentyl- oder Phenylgruppe,

$R_4$ eine Methyl- oder Äthylgruppe und

W eine Carboxy-, Methoxycarbonyl- oder Äthoxycarbonylgruppe darstellen, deren Enantiomere und deren Salze.

4. Phenylessigsäurederivate der allgemeinen Formel I gemäß Anspruch 1, in der

$R_1$ bis $R_4$ wie im Anspruch 3 definiert sind und

W die Carboxygruppe darstellt, deren Enantiomere und deren Salze.

5. 2-Äthoxy-4-[N-(1-(2-piperidino-phenyl)-1-butyl)-aminocarbonylmethyl]-benzoesäure, deren Enantiomere und deren Salze.

6. 2-Äthoxy-4-[N-(1-(2-piperidino-phenyl)-3-methyl-1-butyl)-aminocarbonylmethyl]-benzoesäure, deren Enantiomere und deren Salze.

7. Physiologisch verträgliche Salze der Verbindungen gemäß den Ansprüchen 1 bis 6 mit anorganischen oder organischen Säuren oder auch Basen, sofern diese eine Carboxygruppe enthalten.

8. Arzneimittel, enthaltend eine Verbindung gemäß den Ansprüchen 1 bis 6, in der $R_4$ eine Methyl-, Äthyl- oder Allylgruppe darstellt, oder ein physiologisch verträgliches Salz hiervon neben einem oder mehreren inerten Trägerstoffen und/oder Verdünnungsmitteln.

9. Verwendung der Verbindungen gemäß Anspruch 1 bis 6, in der $R_4$ eine Methyl-, Äthyl- oder Allylgruppe darstellt, oder ein physiologisch verträgliches Salz hiervon zur Herstellung eines Arzneimittels, welches zur Behandlung des Diabetes mellitus geeignet ist.

10. Verfahren zur Herstellung eines Arzneimittels gemäß Anspruch 8, dadurch gekennzeichnet, daß auf nichtchemischem Wege eine Verbindung gemäß den Ansprüchen 1 bis 6, in der $R_4$ eine Methyl-, Äthyl- oder Allylgruppe darstellt, oder ein physiologisch verträgliches Salz hiervon in einen oder mehrere inerte Trägerstoffe und/oder Verdünnungsmittel eingearbeitet wird.

11. Verfahren zur Herstellung von neuen Phenylessigsäurederivaten gemäß den Ansprüchen 1 bis 7, dadurch gekennzeichnet, daß

a) ein Amin der allgemeinen Formel

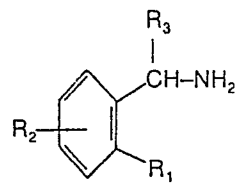

, (II)

in der

$R_1$ bis $R_3$ wie in den Ansprüchen 1 bis 6 definiert sind, mit einer Carbonsäure der allgemeinen Formel

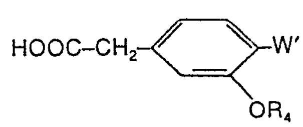

, (III)

in der

$R_4$ wie in den Ansprüchen 1 bis 6 definiert ist und

W' die für W in den Ansprüchen 1 bis 6 erwähnten Bedeutungen besitzt oder eine durch einen Schutzrest geschützte Carboxylgruppe darstellt, oder mit deren gegebenenfalls im Reaktionsgemisch hergestellten reaktionsfähigen Derivaten umgesetzt und erforderlichenfalls ein verwendeter Schutzrest abgespalten wird, oder

b) zur Herstellung einer Verbindung der allgemeinen Formel I, in der W eine Carboxygruppe darstellt, eine Gruppe A in einer Verbindung der allgemeinen Formel

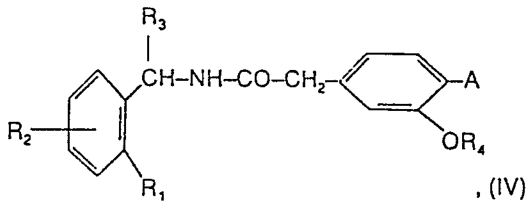

, (IV)

in der

$R_1$ bis $R_4$ wie in den Ansprüchen 1 bis 6 definiert sind und

A eine durch Hydrolyse, Thermolyse oder Hydrogenolyse in eine Carboxygruppe überführbare Gruppe darstellt, in eine Carboxygruppe übergeführt wird,

c) zur Herstellung einer Verbindung der allgemeinen Formel I, in der $R_4$ ein Wasserstoffatom, eine Methyl- oder Äthylgruppe darstellt, eine Verbindung der allgemeinen Formel

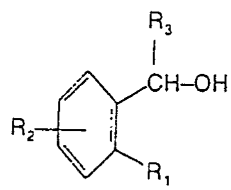

, (V)

in der

$R_1$ bis $R_3$ wie in den Ansprüchen 1 bis 6 definiert sind, mit einer Verbindung der allgemeinen Formel

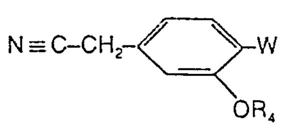

, (VI)

in der

$R_4$ und W wie in den Ansprüchen 1 bis 6 definiert sind, umgesetzt wird, oder

d) zur Herstellung von Verbindungen der allgemeinen Formel I, in der $R_4$ ein Wasserstoffatom darstellt, ein Schutzrest von einer Verbindung der allgemeinen Formel

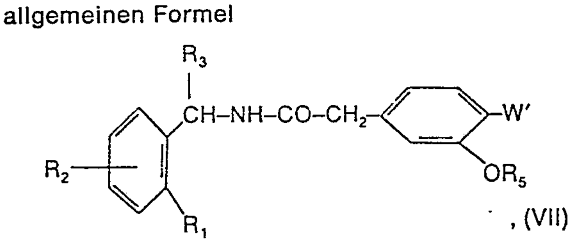

, (VII)

in der

$R_1$ bis $R_3$ wie in den Ansprüchen 1 bis 6 definiert sind,

W' die für W in den Ansprüchen 1 bis 6 erwähnten Bedeutungen besitzt oder eine in eine Carboxygruppe überführbare Gruppe darstellt und

$R_5$ ein Schutzrest für eine Hydroxygruppe darstellt, abgespalten wird, oder

e) zur Herstellung von Verbindungen der allgemeinen Formel I, in der $R_4$ eine Methyl-, Äthyl- oder Allylgruppe darstellt, eine Verbindung der allgemeinen Formel

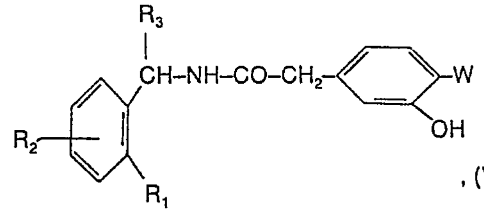

, (VIII)

in der

$R_1$ bis $R_3$ und W wie in den Ansprüchen 1 bis 6 definiert sind, mit einer Verbindung der allgemeinen Formel

$$R_4'-X \qquad (IX)$$

in der

$R_4'$ mit Ausnahme des Wasserstoffatoms die für $R_4$ in den Ansprüchen 1 bis 6 erwähnten Bedeutungen besitzt und

X eine nukleophil austauschbare Gruppe oder auch zusammen mit dem benachbarten Wasserstoffatom eine Diazogruppe, wenn $R_4'$ eine Methyl- oder Äthylgruppe darstellt, bedeuten, umgesetzt und erforderlichenfalls anschließend eine so erhaltene Verbindung hydrolysiert wird, oder

f) eine Verbindung der allgemeinen Formel

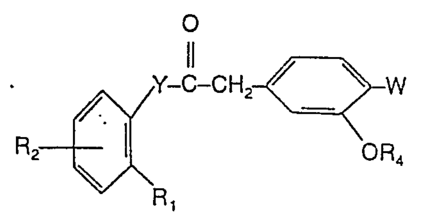

, (X)

in der

$R_1$, $R_2$, $R_4$ und W wie in den Ansprüchen 1 bis 6 definiert sind und

Y eine Gruppe der Formel

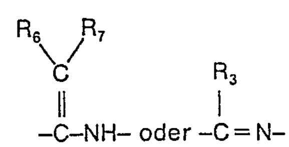

oder

darstellt, wobei $R_3$ wie in den Ansprüchen 1 bis 6 definiert ist und

$R_6$ und $R_7$ zusammen mit dem dazwischen liegenden Kohlenstoffatom eine Alkylidengruppe mit 1 bis 4 Kohlenstoffatomen darstellen,

reduziert wird und

gewünschtenfalls anschließend eine so erhaltene Verbindung der allgemeinen Formel I, in der $R_2$ ein Halogenatom und/oder $R_3$ eine Halogenphenylgruppe und/oder W eine Hydroxymethylgruppe, welche in eine Halogenmethylgruppe überführt wurde, bedeuten, mittels Enthalogenierung in eine entsprechende Verbindung der allgemeinen Formel I, in der $R_2$ ein Wasserstoffatom und/oder $R_3$ eine Phenylgruppe und/oder W die Methylgruppe bedeuten, übergeführt wird, und/oder

eine so erhaltene Verbindung der allgemeinen Formel I, in der W die Carboxygruppe darstellt,

mittels Veresterung in eine entsprechende Verbindung der allgemeinen Formel I, in der W eine Alkoxycarbonyl- oder Phenylalkoxycarbonylgruppe bedeutet, übergeführt wird, und/oder

eine so erhaltene Verbindung der allgemeinen Formel I, in der W eine Carboxy-, Alkoxycarbonyl- oder Phenylalkoxycarbonylgruppe darstellt, mittels Reduktion in eine entsprechende Verbindung der allgemeinen Formel I, in der W eine Formyl- oder Hydroxymethylgruppe darstellt, übergeführt wird, und/oder

eine so erhaltene Verbindung der allgemeinen Formel I, in der W die Hydroxymethylgruppe darstellt, mittels Oxidation in eine entsprechende Verbindung der allgemeinen Formel I, in der W eine Formyl- oder Carboxygruppe darstellt, übergeführt wird, und/oder

eine so erhaltene Verbindung der allgemeinen Formel I, in der W die Carboxygruppe darstellt, mittels Überführung in ein Sulfonsäurehydrazid und anschließende Disproportionierung in eine entsprechende Verbindung der allgemeinen Formel I, in der W die Formylgruppe darstellt, übergeführt wird, und/oder

eine so erhaltene racemische Verbindung der allgemeinen Formel I, in der $R_3$ mit Ausnahme des Wasserstoffatoms wie in den Ansprüchen 1 bis 6 definiert ist, durch Chromatographie an chiralen Phasen in ihre Enantiomeren aufgetrennt wird, und/oder

eine so erhaltene Verbindung der allgemeinen Formel I in ihre Salze, insbesondere in ihre physiologisch verträglichen Salze mit anorganischen oder organischen Säuren oder Basen übergeführt wird.

**Patentansprüche für den Vertragsstaat: AT**

1. Verfahren zur Herstellung von neuen Phenylessigsäurederivaten der allgemeinen Formel

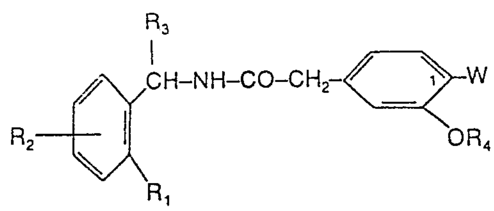

, (I)

in der

$R_1$ eine Pyrrolidino-, Piperidino-, Hexamethylenimino-, Methyl-pyrrolidino-, Dimethyl-pyrrolidino-, 2-Methyl-piperidino-, 3-Methyl-piperidino-, 4-Methylpiperidino-, 3,3-Dimethyl-piperidino-, cis-3,5-Dimethylpiperidino- oder trans-3,5-Dimethyl-piperidinogruppe,

$R_2$ ein Wasserstoff- oder Halogenatom, eine Methyl- oder Methoxygruppe,

$R_3$ ein Wasserstoffatom, eine Alkylgruppe mit 1 bis 4 Kohlenstoffatomen, eine n-Pentyl- oder 3-Methyl-n-butylgruppe oder eine gegebenenfalls durch ein Halogenatom, eine Methyl- oder Methoxygruppe substituierte Phenylgruppe,

$R_4$ ein Wasserstoffatom, eine Methyl-, Äthyl- oder Allylgruppe und

W eine Methyl-, Hydroxymethyl-, Formyl-, Carboxy- oder Alkoxycarbonylgruppe mit insgesamt 2 bis 5 Kohlenstoffatomen, wobei der Alkylteil der Alkoxygruppe durch eine Phenylgruppe substituiert sein kann, bedeuten, von deren Enantiomeren und von deren Salzen, dadurch gekennzeichnet, daß

a) ein Amin der allgemeinen Formel

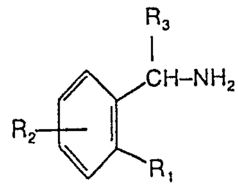

, (II)

in der

$R_1$ bis $R_3$ wie eingangs definiert sind, mit einer Carbonsäure der allgemeinen Formel

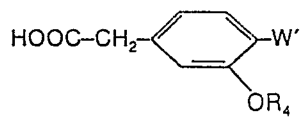

, (III)

in der

$R_4$ wie eingangs definiert ist und

W′ die für W eingangs erwähnten Bedeutungen besitzt oder eine durch einen Schutzrest geschützte Carboxylgruppe darstellt,

oder mit deren gegebenenfalls im Reaktionsgemisch hergestellten reaktionsfähigen Derivaten umgesetzt und erforderlichenfalls ein verwendeter Schutzrest abgespalten wird, oder

b) zur Herstellung einer Verbindung der allgemeinen Formel I, in der W eine Carboxygruppe darstellt, eine Gruppe A in einer Verbindung der allgemeinen Formel

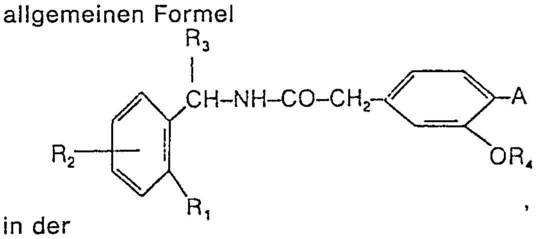

, (IV)

in der

$R_1$ bis $R_4$ wie eingangs definiert sind und

A eine durch Hydrolyse, Thermolyse oder Hydrogenolyse in eine Carboxygruppe überführbare Gruppe darstellt, in eine Carboxygruppe übergeführt wird,

c) zur Herstellung einer Verbindung der allgemeinen Formel I, in der $R_4$ ein Wasserstoffatom, eine Methyl- oder Äthylgruppe darstellt, eine Verbindung der allgemeinen Formel

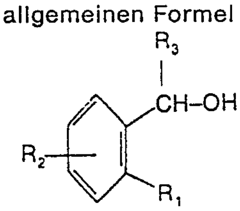

, (V)

in der

$R_1$ bis $R_3$ wie eingangs definiert sind, mit einer Verbindung der allgemeinen Formel

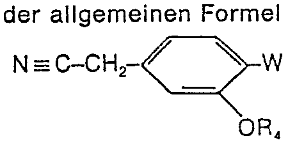

, (VI)

in der

$R_4$ und W wie eingangs definiert sind, umgesetzt wird, oder

d) zur Herstellung von Verbindungen der allgemeinen Formel I, in der $R_4$ ein Wasserstoffatom darstellt, ein Schutzrest von einer Verbindung der allgemeinen Formel

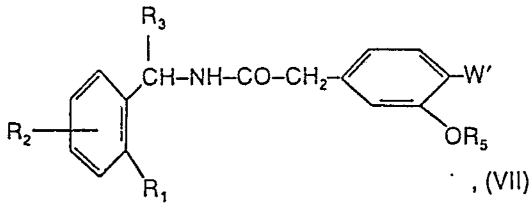

, (VII)

in der

$R_1$ bis $R_3$ wie eingangs definiert sind,

W' die für W eingangs erwähnten Bedeutungen besitzt oder eine in eine Carboxygruppe überführbare Gruppe darstellt und

$R_5$ ein Schutzrest für eine Hydroxygruppe darstellt, abgespalten wird, oder

e) zur Herstellung von Verbindungen der allgemeinen Formel I, in der $R_4$ eine Methyl-, Äthyl- oder Allylgruppe darstellt, eine Verbindung der allgemeinen Formel

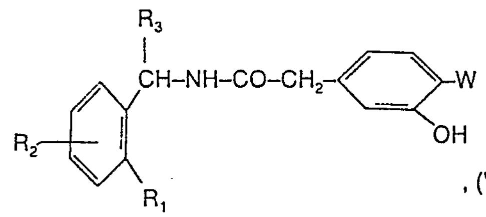

, (VIII)

in der

$R_1$ bis $R_3$ und W wie eingangs definiert sind, mit einer Verbindung der allgemeinen Formel

$$R_4'-X, \qquad (IX)$$

in der

$R_4'$ mit Ausnahme des Wasserstoffatoms die für $R_4$ eingangs erwähnten Bedeutungen besitzt und

X eine nukleophil austauschbare Gruppe oder auch zusammen mit dem benachbarten Wasserstoffatom eine Diazogruppe, wenn $R_4'$ eine Methyl- oder Äthylgruppe darstellt, bedeuten, umgesetzt und erforderlichenfalls anschließend eine so erhaltene Verbindung hydrolysiert wird, oder

f) eine Verbindung der allgemeinen Formel

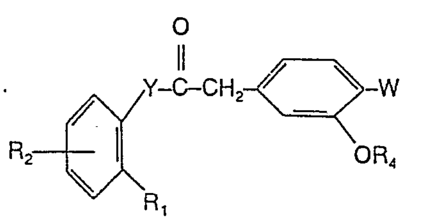

, (X)

in der

$R_1$, $R_2$, $R_4$ und W wie eingangs definiert sind und Y eine Gruppe der Formel

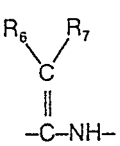 oder 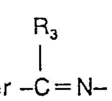

darstellt, wobei $R_3$ wie eingangs definiert ist und $R_6$ und $R_7$ zusammen mit dem dazwischen liegenden Kohlenstoffatom eine Alkylidengruppe mit 1 bis 4 Kohlenstoffatomen darstellt,

. reduziert wird und

gewünschtenfalls anschließend eine so erhaltene Verbindung der allgemeinen Formel I, in der $R_2$ ein Halogenatom und/oder $R_3$ eine Halogenphenylgruppe und/oder W eine Hydroxymethylgruppe, welche in eine Halogenmethylgruppe überführt wurde, bedeuten, mittels Enthalogenierung in eine entsprechende Verbindung der allgemeinen Formel I, in der $R_2$ ein Wasserstoffatom und/oder $R_3$ eine Phenylgruppe und/oder W die Methylgruppe bedeuten, übergeführt wird, und/oder

eine so erhaltene Verbindung der allgemeinen Formel I, in der W die Carboxygruppe darstellt, mittels Veresterung in eine entsprechende Verbindung der allgemeinen Formel I, in der W eine Alkoxycarbonyl- oder Phenylalkoxycarbonylgruppe bedeutet, übergeführt wird, und/oder

eine so erhaltene Verbindung der allgemeinen Formel I, in der W eine Carboxy-, Alkoxycarbonyl- oder Phenylalkoxycarbonylgruppe darstellt, mittels Reduktion in eine entsprechende Verbindung der allgemeinen Formel I, in der W eine Formyl- oder Hydroxymethylgruppe darstellt, übergeführt wird, und/oder

eine so erhaltene Verbindung der allgemeinen Formel I, in der W die Hydroxymethylgruppe darstellt, mittels Oxidation in eine entsprechende Verbindung der allgemeinen Formel I, in der W eine Formyl- oder Carboxygruppe darstellt, übergeführt wird, und/oder

eine so erhaltene Verbindung der allgemeinen Formel I, in der W die Carboxygruppe darstellt, mittels Überführung in ein Sulfonsäurehydrazid und anschließende Disproportionierung in eine entsprechende Verbindung der allgemeinen Formel I, in der W die Formylgruppe darstellt, übergeführt wird, und/oder

eine so erhaltene racemische Verbindung der allgemeinen Formel I, in der $R_3$ mit Ausnahme des Wasserstoffatoms wie eingangs definiert ist, durch Chromatographie an chiralen Phasen in ihre Enantiomeren aufgetrennt wird, und/oder

eine so erhaltene Verbindung der allgemeinen Formel I in ihre Salze, insbesondere in ihre physiologisch verträglichen Salze mit anorganischen oder organischen Säuren oder Basen übergeführt wird.

2. Verfahren nach Anspruch 1, dadurch gekennzeichnet, daß die Umsetzung in einem Lösungsmittel durchgeführt wird.

3. Verfahren nach den Ansprüchen 1a und 2, dadurch gekennzeichnet, daß die Umsetzung in Gegenwart eines die Säure aktivierenden Mittels, eines wasserentziehenden Mittels oder in Gegenwart eines das Amin aktivierenden Mittels durchgeführt wird.

4. Verfahren nach den Ansprüchen 1a und 3, dadurch gekennzeichnet, daß die Umsetzung bei Temperaturen zwischen −25 und 250 °C, vorzugsweise bei Temperaturen zwischen −10 °C und der

Siedetemperatur des verwendeten Lösungsmittels, durchgeführt wird.

5. Verfahren nach den Ansprüchen 1b und 2, dadurch gekennzeichnet, daß, wenn A die Nitril- oder Aminocarbonylgruppe darstellt, die Umsetzung in Gegenwart eines Nitrits, z.B. Natriumnitrit, und in Gegenwart einer Säure wie Schwefelsäure bei Temperaturen zwischen 0 und 50 °C oder mittels 100%iger Phosphorsäure bei Temperaturen zwischen 100 und 180 °C, vorzugsweise bei Temperaturen zwischen 120 und 160 °C, durchgeführt wird.

6. Verfahren nach den Ansprüchen 1c und 2, dadurch gekennzeichnet, daß die Umsetzung bei Temperaturen zwischen 0 und 150 °C, vorzugsweise bei Temperaturen zwischen 20 und 100 °C, durchgeführt wird.

7. Verfahren nach den Ansprüchen 1d und 2, dadurch gekennzeichnet, daß die Umsetzung in Gegenwart einer Säure bei Temperaturen zwischen 0 und 150 °C oder mit Bortribromid bei Temperaturen zwischen −78 und 20 °C durchgeführt wird.

8. Verfahren nach den Ansprüchen 1e und 2, dadurch gekennzeichnet, daß die Umsetzung bei Temperaturen zwischen 0 und 100 °C, vorzugsweise bei Temperaturen zwischen 20 und 50 °C, durchgeführt wird.

9. Verfahren nach den Ansprüchen 1f und 2, dadurch gekennzeichnet, daß die Reduktion mit Wasserstoff in Gegenwart eines Hydrierungskatalysators bei Temperaturen zwischen 0 und 100 °C, vorzugsweise jedoch bei Temperaturen zwischen 20 und 50 °C, durchgeführt wird.

10. Verfahren zur Herstellung eines Arzneimittel zur Behandlung des Diabetes mellitus, dadurch gekennzeichnet, daß eine Verbindung der allgemeinen Formel I gemäß Anspruch 1, in der $R_4$ eine Methyl-, Äthyl- oder Allylgruppe darstellt, oder ein physiologisch verträgliches Salz hiervon in einen oder mehrere inerte Trägerstoffe und/oder Verdünnungsmitteln eingearbeitet wird.

**Claims for the contracting states: BE, CH, DE, FR, GB, IT, LI, LU, NL, SE**

1. Phenylacetic acid derivatives of general formula

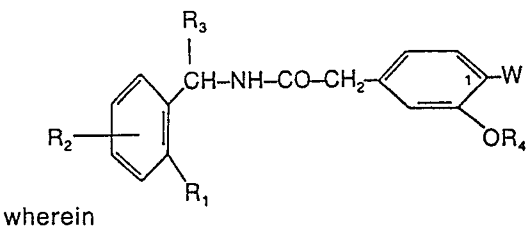

, (I)

wherein

$R_1$ represents a pyrrolidino, piperidino, hexamethyleneimino, methyl-pyrrolidino, dimethyl-pyrrolidino, 2-methyl-piperidino, 3-methyl-piperidino, 4-methyl-piperidino, 3,3-dimethyl-piperidino, cis-3,5-dimethyl-piperidino or trans-3,5-dimethyl-piperidino group,

$R_2$ represents a hydrogen or halogen atom or a methyl or methoxy group,

$R_3$ represents a hydrogen atom, an alkyl group with 1 to 4 carbon atoms, an n-pentyl or 3-methyl-n-butyl group or a phenyl group optionally substituted by a halogen atom or by a methyl or methoxy group,

$R_4$ represents a hydrogen atom, a methyl, ethyl or allyl group and

W represents a methyl, hydroxymethyl, formyl, carboxy or alkoxycarbonyl group with a total of 2 to 5 carbon atoms, whilst the alkyl part of the alkoxy group may be substituted by a phenyl group,

the enantiomers and salts thereof.

2. Phenylacetic acid derivatives of general formula I as claimed in claim 1, wherein

$R_3$ and $R_4$ are defined as in claim 1,

$R_1$ represents a pyrrolidino, piperidino, 4-methyl-piperidino, 3-methyl-piperidino, 3,3-dimethyl-piperidino, 3,5-dimethyl-piperidino or hexamethyleneimino group,

$R_2$ represents a hydrogen, fluorine or chlorine atom or a methyl or methoxy group and

W represents a carboxy, methyl, hydroxymethyl, formyl or benzyloxycarbonyl group or an alkoxycarbonyl group with a total of 2 to 5 carbon atoms, the enantiomers and salts thereof.

3. Phenylacetic acid derivatives of general formula I as claimed in claim 1, wherein

$R_1$ represents a piperidino group,

$R_2$ represents a hydrogen, fluorine or chlorine atom,

$R_3$ represents a methyl, ethyl, n-propyl, n-butyl, isobutyl, n-pentyl or phenyl group,

$R_4$ represents a methyl or ethyl group and

W represents a carboxy, methoxycarbonyl or ethoxycarbonyl group,

the enantiomers and salts thereof.

4. Phenylacetic acid derivatives of general formula I as claimed in claim 1, wherein

$R_1$ to $R_4$ are defined as in claim 3 and

W represents the carboxy group, the enantiomers and salts thereof.

5. 2-Ethoxy-4-[N-(1-(2-piperidino-phenyl)-1-butyl)-aminocarbonylmethyl]-benzoic-acid, the enantiomers and salts thereof.

6. 2-Ethoxy-4-[N-(1-(2-piperidino-phenyl)-3-methyl-1-butyl)-aminocarbonylmethyl]-benzoic acid, the enantiomers and salts thereof.

7. Physiologically acceptable salts of the compounds as claimed in claims 1 to 6 with inorganic or organic acids or bases, if the latter contain a carboxy group.

8. Pharmaceutical compositions containing a compound as claimed in claims 1 to 6, wherein $R_4$ represents a methyl, ethyl or allyl group or a physiologically acceptable salt thereof together with one or more inert carriers and/or diluents.

9. Use of the compounds as claimed in claims 1 to 6, wherein $R_4$ represents a methyl, ethyl or allyl group, or a physiologically acceptable salt thereof for the preparation of a pharmaceutical composition which is suitable for treating Diabetes mellitus.

10. Process for preparing a pharmaceutical composition as claimed in claim 8, characterised

in that, by a non-chemical method, a compound as claimed in claims 1 to 6, wherein $R_4$ represents a methyl, ethyl or allyl group, or a physiologically acceptable salt thereof is incorporated in one or more inert carriers and/or diluents.

11. Process for preparing new phenylacetic acid derivatives as claimed in claims 1 to 7, characterised in that

a) an amine of general formula

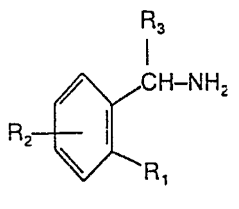

(II)

wherein

$R_1$ to $R_3$ are defined as in claims 1 to 6 is acylated with a carboxylic acid of general formula

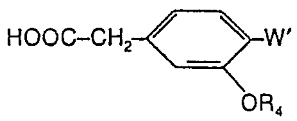

(III)

wherein

$R_4$ is defined as in claims 1 to 6 and

W' has the meanings given for W in claims 1 to 6 or represents a carboxy group protected by a protecting group,

or with the reactive derivatives thereof, which may optionally be prepared in the reaction mixture, and if necessary any protecting group used is split off, or

b) in order to prepare a compound of general formula I wherein W represents a carboxy group, a group A in a compound of general formula

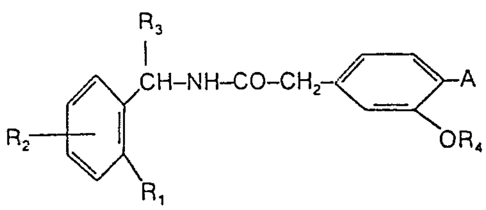

(IV)

wherein

$R_1$ to $R_4$ are defined as in claims 1 to 6 and

A represents a group which may be converted into a carboxy group by hydrolysis, thermolysis or hydrogenolysis,

is converted into a carboxy group,

c) in order to prepare a compound of general formula I wherein $R_4$ represents a hydrogen atom or a methyl or ethyl group, a compound of general formula

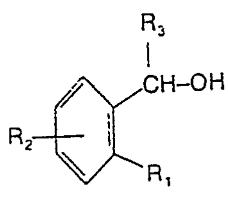

(V)

wherein

$R_1$ to $R_3$ are defined as in claims 1 to 6 is reacted with a compound of general formula

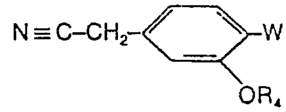

(VI)

wherein

$R_4$ and W are defined as in claims 1 to 6, or

d) in order to prepare compounds of general formula I wherein $R_4$ represents a hydrogen atom, a protecting group is cleaved from a compound of general formula

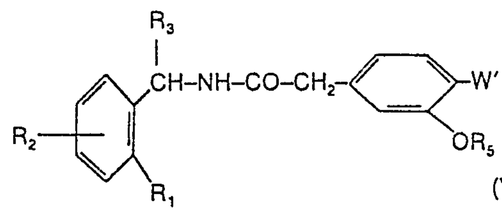

(VII)

wherein

$R_1$ to $R_3$ are defined as in claims 1 to 6,

W' has the meanings given for W in claims 1 to 6 or represents a group which can be converted into a carboxy group and

$R_5$ represents a protecting group for a hydroxy group or

e) in order to prepare compounds of general formula I wherein $R_4$ represents a methyl, ethyl or allyl group, a compound of general formula

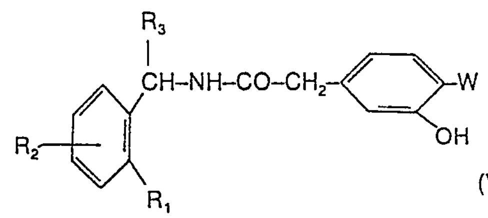

(VIII)

wherein

$R_1$ to $R_3$ and W are defined as in claims 1 to 6, is reacted with a compound of general formula

$$R_4'-X \qquad (IX)$$

wherein

$R_4'$ has the meanings given for $R_4$ in claims 1 to 6, with the exception of the hydrogen atom, and

X represents a nucleophilically exchangeable group or, together with the adjacent hydrogen atom, represents a diazo group if $R_4'$ represents a methyl or ethyl group, and subsequently, if necessary, a compound thus obtained is hydrolysed, or

f) a compound of general formula

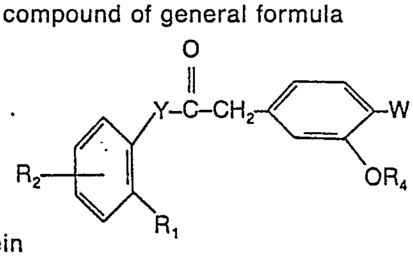

(X)

wherein

$R_1$, $R_2$, $R_4$ and W are defined as in claims 1 to 6 and

Y represents a group of formula

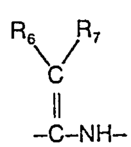

or

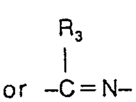

wherein $R_3$ is defined as in claims 1 to 6 and $R_6$ and $R_7$ together with the carbon atom between them represent an alkylidene group with 1 to 4 carbon atoms,
is reduced and

subsequently, if desired, a compound of general formula I thus obtained wherein $R_2$ represents a halogen atom and/or $R_3$ represents a halophenyl group and/or W represents a hydroxymethyl group which has been converted into a halomethyl group, is converted by dehalogenation into a corresponding compound of general formula I wherein $R_2$ represents a hydrogen atom and/or $R_3$ represents a phenyl group and/or W represents a methyl group, and/or

a compound of general formula I thus obtained wherein W represents the carboxy group is converted by esterification into a corresponding compound of general formula I wherein W represents an alkoxycarbonyl or phenylalkoxycarbonyl group, and/or

a compound of general formula I thus obtained wherein W represents a carboxy, alkoxycarbonyl or phenylalkoxycarbonyl group is converted by reduction into a corresponding compound of general formula I wherein W represents a formyl or hydroxymethyl group, and/or

a compound of general formula I thus obtained wherein W represents a hydroxymethyl group is converted by oxidation into a corresponding compound of general formula I wherein W represents a formyl or carboxy group, and/or

a compound of general formula I thus obtained wherein W represents a carboxy group is converted by conversion into a sulphonic acid hydrazide and subsequent disproportionation into a corresponding compound of general formula I wherein W represents a formyl group, and/or

a racemic compound of general formula I thus obtained wherein $R_3$ has the meanings given in claims 1 to 6, with the exception of the hydrogen atom, is resolved by chromatography on chiral phases into the enantiomers thereof,
and/or

a compound of general formula I thus obtained is converted into the salts thereof, more particularly the physiologically acceptable salts thereof with inorganic or organic acids or bases.

**Claims for the contracting state: AT**

1. Process for preparing new phenylacetic acid derivatives of general formula

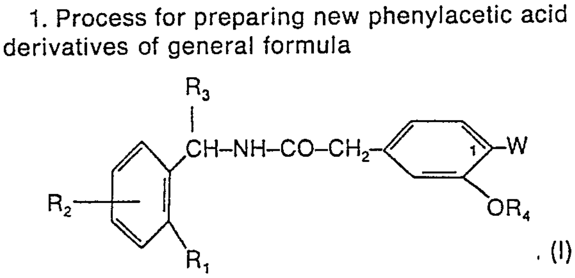

, (I)

wherein

$R_1$ represents a
pyrrolidino, piperidino, hexamethyleneimino, methyl-pyrrolidino, dimethyl-pyrrolidino, 2-methyl-piperidino, 3-methyl-piperidino, 4-methyl-piperidino, 3,3-dimethyl-piperidino, cis-3,5-dimethyl-piperidino or trans-3,5-dimethyl-piperidino group,

$R_2$ represents a hydrogen or halogen atom or a methyl or methoxy group,

$R_3$ represents a hydrogen atom, an alkyl group with 1 to 4 carbon atoms, an n-pentyl or 3-methyl-n-butyl group or a phenyl group optionally substituted by a halogen atom or by a methyl or methoxy group,

$R_4$ represents a hydrogen atom, a methyl, ethyl or allyl group and

W represents a methyl, hydroxymethyl, formyl, carboxy or alkoxycarbonyl group with a total of 2 to 5 carbon atoms, whilst the alkyl part of the alkoxy group may be substituted by a phenyl group, the enantiomers and salts thereof, characterised in that

a) an amine of general formula

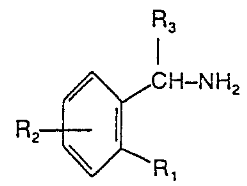

(II)

wherein

$R_1$ to $R_3$ are defined as hereinbefore is acylated with a carboxylic acid of general formula

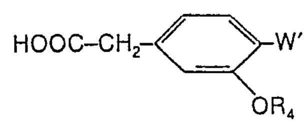

(III)

wherein

$R_4$ is defined as hereinbefore and

W' has the meanings given for W hereinbefore or represents a carboxy group protected by a protecting group,
or with the reactive derivatives thereof, which may optionally be prepared in the reaction mixture, and if necessary any protecting group used is split off, or

b) in order to prepare a compound of general formula I wherein W represents a carboxy group, a group A in a compound of general formula

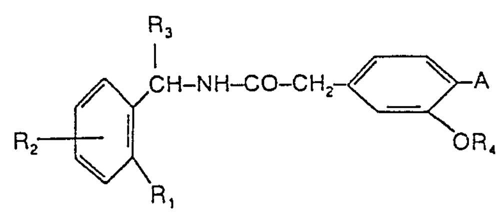

(IV)

wherein

$R_1$ to $R_4$ are defined as hereinbefore and

A represents a group which may be converted into a carboxy group by hydrolysis, thermolysis or hydrogenolysis,
is converted into a carboxy group,

c) in order to prepare a compound of general formula I wherein $R_4$ represents a hydrogen atom or a methyl or ethyl group, a compound of general formula

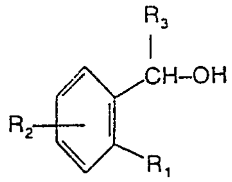

(V)

wherein

$R_1$ to $R_3$ are defined as hereinbefore is reacted with a compound of general formula

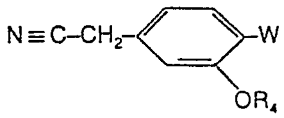

(VI)

wherein

$R_4$ and W are defined as hereinbefore, or

d) in order to prepare compounds of general formula I wherein $R_4$ represents a hydrogen atom, a protecting group is cleaved from a compound of general formula

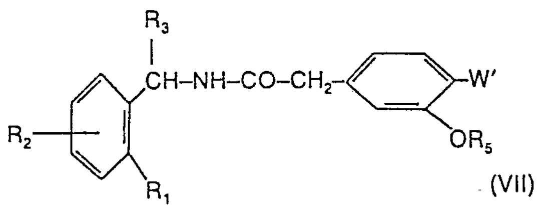

(VII)

wherein

$R_1$ to $R_3$ are defined as hereinbefore,

W' has the meanings given for W hereinbefore or represents a group which can be converted into a carboxy group and

$R_5$ represents a protecting group for a hydroxy group or

e) in order to prepare compounds of general formula I wherein $R_4$ represents a methyl, ethyl or allyl group, a compound of general formula

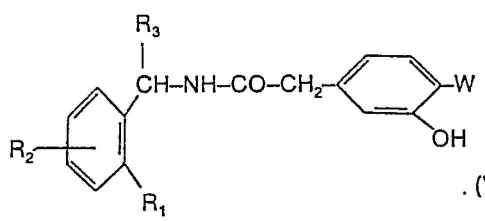

. (VIII)

wherein

$R_1$ to $R_3$ and W are defined as hereinbefore, is reacted with a compound of general formula

$$R_4'\text{–}X \qquad \text{(IX)}$$

wherein

$R_4'$ has the meanings given for $R_4$ hereinbefore, with the exception of the hydrogen atom, and

X represents a nucleophilically exchangeable group or, together with the adjacent hydrogen atom, represents a diazo group if $R_4'$ represents a methyl or ethyl group, and subsequently, if necessary, a compound thus obtained is hydrolysed, or

f) a compound of general formula

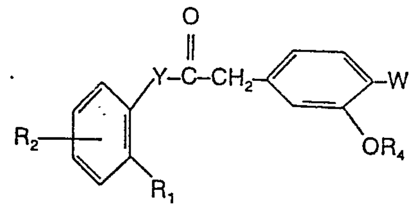

(X)

wherein

$R_1$, $R_2$, $R_4$ and W are defined as hereinbefore and Y represents a group of formula

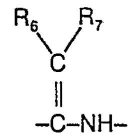

or

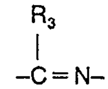

wherein $R_3$ is defined as hereinbefore and $R_6$ and $R_7$ together with the carbon atom between them represent an alkylidene group with 1 to 4 carbon atoms,

is reduced and

subsequently, if desired, a compound of general formula I thus obtained wherein $R_2$ represents a halogen atom and/or $R_3$ represents a halophenyl group and/or W represents a hydroxymethyl group which has been converted into a halomethyl group, is converted by dehalogenation into a corresponding compound of general formula I wherein $R_2$ represents a hydrogen atom and/or $R_3$ represents a phenyl group and/or W represents a methyl group, and/or

a compound of general formula I thus obtained wherein W represents the carboxy group is converted by esterification into a corresponding compound of general formula I wherein W represents an alkoxycarbonyl or phenylalkoxycarbonyl group, and/or

a compound of general formula I thus obtained wherein W represents a carboxy, alkoxycarbonyl or phenylalkoxycarbonyl group is converted by reduction into a corresponding compound of general formula I wherein W represents a formyl or hydroxymethyl group, and/or

a compound of general formula I thus obtained wherein W represents a hydroxymethyl group is converted by oxidation into a corresponding compound of general formula I wherein W represents a formyl or carboxy group, and/or

a compound of general formula I thus obtained wherein W represents a carboxy group is converted by conversion into a sulphonic acid hydrazide and subsequent disproportionation into a corresponding compound of general formula I wherein W represents a formyl group, and/or

a racemic compound of general formula I thus obtained wherein $R_3$ has the meanings given hereinbefore, with the exception of the hydrogen atom, is resolved by chromatography on chiral phases into the enantiomers thereof,

and/or

a compound of general formula I thus obtained is converted into the salts thereof, more particularly the physiologically acceptable salts thereof with inorganic or organic acids or bases.

2. Process as claimed in claim 1, characterised in that the reaction is carried out in a solvent.

3. Process as claimed in claims 1a and 2, characterised in that the reaction is carried out in the presence of an acid-activating or a dehydrating agent, or in the presence of an agent which activates the amine.

4. Process as claimed in claims 1a and 3, characterised in that the reaction is carried out at temperatures of between −25 and 250 °C, preferably at temperatures of between −10 °C and the boiling temperature of the solvent used.

5. Process as claimed in claims 1b and 2, characterised in that, if A represents the nitrile or aminocarbonyl group, the reaction is carried out in the presence of a nitrite, e.g. sodium nitrite, and in the presence of an acid such as sulphuric acid at temperatures of between 0 and 50 °C or using 100% phosphoric acid at temperatures of between 100 and 180 °C, preferably at temperatures of between 120 and 160 °C.

6. Process as claimed in claims 1c and 2, characterised in that the reaction is carried out at temperatures of between 0 and 150 °C, preferably at temperatures of between 20 and 100 °C.

7. Process as claimed in claims 1d and 2, characterised in that the reaction is carried out in the presence of an acid at temperatures of between 0 and 150 °C or with boron tribromide at temperatures of between −78 and 20 °C.

8. Process as claimed in claims 1e and 2, characterised in that the reaction is carried out at temperatures of between 0 and 100 °C, preferably at temperatures of between 20 and 50 °C.

9. Process as claimed in claims 1f and 2, characterised in that the reduction is carried out with hydrogen in the presence of a hydrogenation catalyst at temperatures of between 0 and 100 °C, but preferably at temperatures of between 20 and 50 °C.

10. Process for preparing a pharmaceutical composition for the treatment of Diabetes mellitus, characterised in that a compound of general formula I as claimed in claim 1, wherein $R_4$ represents a methyl, ethyl or allyl group, or a physiologically acceptable salt thereof is incorporated in one or more inert carriers and/or diluents.

**Revendications pour les Etats contractants: BE, CH, DE, FR, GB, IT, LI, LU, NL, SE**

1. Dérivés de l'acide phénylacétique de formule générale

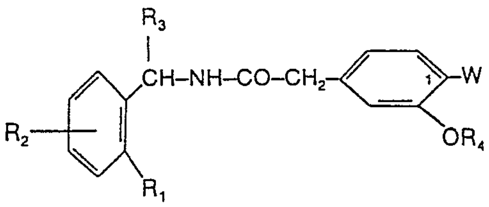

, (I)

dans laquelle

$R_1$ représente un groupe pyrrolidino, pipéridino, hexaméthylènimino, méthyl-pyrrolidino, diméthyl-pyrrolidino, 2-méthyl-pipéridino, 3-méthyl-pipéridino, 4-méthyl-pipéridino, 3,3-diméthyl-pipéridino, cis-3,5-diméthyl-pipéridino ou trans-3,5-diméthyl-pipéridino,

$R_2$ représente un atome d'hydrogène ou d'halogène, un groupe méthyle ou méthoxy,

$R_3$ représente un atome d'hydrogène, un groupe alcoyle avec 1 à 4 atomes de carbone, un groupe n-pentyle ou 3-méthyl-n-butyle ou un groupe phényle éventuellement substitué par un atome d'halogène, un groupe méthyle ou méthoxy,

$R_4$ représente un atome d'hydrogène, un groupe méthyle, éthyle ou allyle et

W représente un groupe méthyle, hydroxyméthyle, formyle, carboxy ou alcoxycarbonyle avec en tout 2 à 5 atomes de carbone, la partie alcoyle du groupe alcoxy pouvant être substituée par un groupe phényle, leurs énantiomères et leurs sels.

2. Dérivés de l'acide phénylacétique de formule générale I, selon la revendication 1, dans laquelle

$R_3$ et $R_4$ sont définis comme dans la revendication 1,

$R_1$ représente un groupe pyrrolidino, pipéridino, 4-méthyl-pipéridino, 3-méthyl-pipéridino, 3,3-diméthyl-pipéridino, 3,5-diméthyl-pipéridino ou hexaméthylènimino,

$R_2$ représente un atome d'hydrogène, de fluor ou de chlore, un groupe méthyle ou méthoxy et

W représente un groupe carboxy, méthyle, hydroxyméthyle, formyle ou benzyloxycarbonyle ou un groupe alcoxycarbonyle avec en tout 2 à 5 atomes de carbone, leurs énantiomères et leurs sels.

3. Dérivés de l'acide phénylacétique de formule générale I selon la revendication 1, dans laquelle

$R_1$ représente un groupe pipéridino,

$R_2$ représente un atome d'hydrogène, de fluor ou de chlore,

$R_3$ représente un groupe méthyle, éthyle, n-propyle, n-butyle, isobutyle, n-pentyle ou phényle,

$R_4$ représente un groupe méthyle ou éthyle et

W représente un groupe carboxy, méthoxycarbonyle ou éthoxycarbonyle, leurs énantiomères et leurs sels.

4. Dérivés de l'acide phénylacétique de formule générale I selon la revendication 1, dans laquelle

$R_1$ à $R_4$ sont définis comme dans la revendication 3 et

W représente le groupe carboxy, leurs énantiomères et leurs sels.

5. L'acide 2-éthoxy-4-[N-(1-(2-pipéridino-phényl)-1-butyl)-amino-carbonylméthyl]-benzoïque, ses énantiomères et ses sels.

6. L'acide 2-éthoxy-4-[N-(1-(2-pipéridino-phényl)-3-méthyl-1-butyl)-aminocarbonyl-méthyl]-benzoïque, ses énantiomères et ses sels.

7. Sels physiologiquement supportables des composés selon les revendications 1 à 6 avec des acides minéraux ou organiques ou également des bases, dans la mesure où ils contiennent un groupe carboxy.

8. Médicament contenant un composé selon les revendications 1 à 6, dans lequel $R_4$ représente un groupe méthyle, éthyle ou allyle, ou un sel physiologiquement supportable de celui-ci conjointement à un ou plusieurs excipients et/ou diluants inertes.

9. Utilisation des composés selon la revendication 1 à 6, dans lequel $R_4$ représente un groupe méthyle, éthyle ou allyle, ou un sel physiologiquement supportable de celui-ci pour la fabrication d'un médicament, lequel convient au traitement du diabète mellitus.

10. Procédé pour la fabrication d'un médicament selon la revendication 8, caractérisé en ce qu'on introduit, par voie non chimique un composé selon les revendications 1 à 6, dans lequel $R_4$ représente un groupe méthyle, éthyle ou allyle, ou un sel physiologiquement supportable de celui-ci dans un ou plusieurs excipients et/ou diluants inertes.

11. Procédé pour la préparation des nouveaux dérivés de l'acide phénylacétique selon les revendications 1 à 7, caractérisé en ce que

a) on fait réagir une amine de formule générale

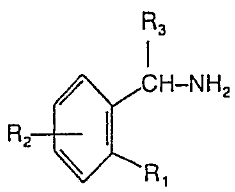

, (II)

dans laquelle

$R_1$ à $R_3$ sont définis comme dans les revendications 1 à 6, avec un acide carboxylique de formule générale

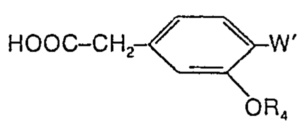

, (III)

dans laquelle

$R_4$ est défini comme dans les revendications 1 à 6 et

W' possède les significations mentionnées pour W dans les revendications 1 à 6 ou représente un groupe carboxyle protégé par un radical protecteur,

ou avec leurs dérivés réactifs, éventuellement préparés dans le mélange réactionnel et si nécessaire, on clive un radical protecteur utilisé, ou

b) pour la préparation d'un composé de formule générale I dans laquelle W représente un groupe carboxy, on transforme en un groupe carboxy un groupe A dans un composé de formule générale

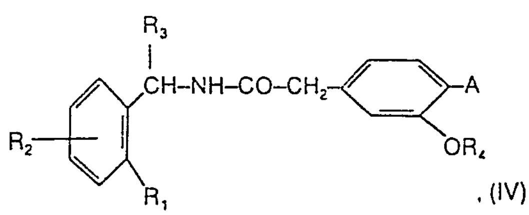

, (IV)

dans laquelle

$R_1$ à $R_4$ sont définis comme dans les revendications 1 à 6 et

A représente un groupe transformable en un groupe carboxy par hydrolyse, thermolyse ou hydrogénolyse,

c) pour la préparation d'un composé de formule générale I dans laquelle $R_4$ représente un atome d'hydrogène, un groupe méthyle ou éthyle, on fait réagir un composé de formule générale

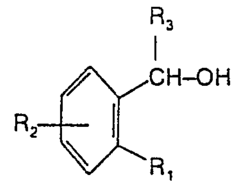

, (V)

dans laquelle

$R_1$ à $R_3$ sont définis comme dans les revendications 1 à 6, avec un composé de formule générale

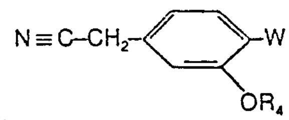

, (VI)

dans laquelle

$R_4$ et W sont définis comme dans les revendications 1 à 6, ou

d) pour la préparation de composés de formule générale I dans laquelle $R_4$ représente un atome d'hydrogène, on clive un radical protecteur à partir d'un composé de formule générale

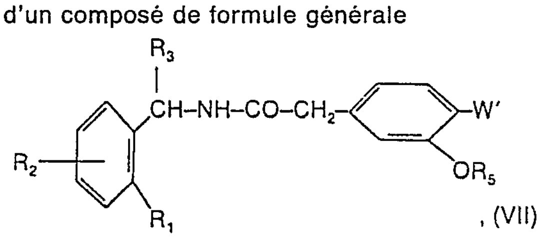

, (VII)

dans laquelle

$R_1$ à $R_3$ sont définis comme dans les revendications 1 à 6,

W' possède les significations mentionnées pour W dans les revendications 1 à 6 ou représente un groupe transformable en un groupe carboxy et

$R_5$ représente un radical protecteur pour un groupe hydroxy, ou

e) pour la préparation de composés de formule générale I dans laquelle $R_4$ représente un groupe méthyle, éthyle ou allyle, on fait réagir un composé de formule générale

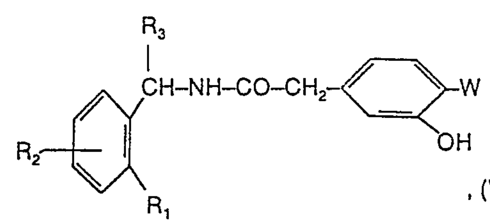

, (VIII)

dans laquelle

$R_1$ à $R_3$ et W sont définis comme dans les revendications 1 à 6, avec un composé de formule générale

$$R_4'-X \qquad (IX)$$

dans laquelle

$R_4'$ possède les significations mentionnées pour $R_4$ dans les revendications 1 à 6, à l'exception de l'atome d'hydrogène et

X représente un groupe échangeable de façon nucléophile ou également ensemble avec l'atome d'hydrogène voisin, un groupe diazo, lorsque $R_4'$ représente un groupe méthyle ou éthyle, et si

nécessaire on hydrolyse ensuite un composé ainsi obtenu, ou

f) un composé de formule générale

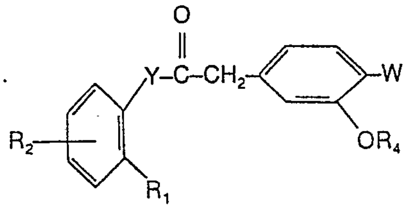

, (X)

dans laquelle

$R_1$, $R_2$, $R_4$ et W sont définis comme dans les revendications 1 à 6 et

Y représente un groupe de formule

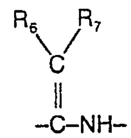 ou 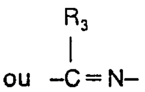 ,

$R_3$ y étant défini comme dans les revendications 1 à 6 et

$R_6$ et $R_7$, ensemble avec l'atome de carbone intermédiaire, y représentant un groupe alcoylidène avec 1 à 4 atomes de carbone,

est réduit, et

si on le désire, on transforme ensuite un composé ainsi obtenu de formule générale I dans laquelle $R_2$ représente un atome d'halogène et/ou $R_3$ représente un groupe halogénophényle et/ou W représente un groupe hydroxyméthyle qui a été transformé en un groupe halogénométhyle, au moyen d'une déshalogénation en un composé correspondant de formule générale I dans laquelle $R_2$ représente un atome d'hydrogène et/ou $R_3$ représente un groupe phényle et/ou W représente le groupe méthyle, et/ou

on transforme un composé ainsi obtenu de formule générale I dans laquelle W représente le groupe carboxy, par estérification en un composé correspondant de formule générale I dans laquelle W représente un groupe alcoxycarbonyle ou phénylalcoxycarbonyle, et/ou

on transforme un composé ainsi obtenu de formule générale I dans laquelle W représente un groupe carboxy, alcoxycarbonyle ou phénylalcoxycarbonyle, par réduction en un composé correspondant de formule générale I dans laquelle W représente un groupe formyle ou hydroxyméthyle, et/ou

on transforme un composé ainsi obtenu de formule générale I dans laquelle W représente le groupe hydroxyméthyle, par oxydation en un composé correspondant de formule générale I, dans laquelle W représente un groupe formyle ou carboxy, et/ou

on transforme un composé ainsi obtenu de formule générale I dans laquelle W représente le groupe carboxy, par transformation en un hydrazide d'acide sulfonique et dismutation subséquente en un composé correspondant de formule générale I, dans laquelle W représente le groupe formyle, et/ou

on sépare un composé racémique ainsi obtenu de formule générale I dans laquelle $R_3$ est défini comme dans les revendications 1 à 6, à l'exception de l'atome d'hydrogène, par chromatographie sur des phases chirales en ses énantiomères, et/ou

on transforme un composé ainsi obtenu de formule générale I en ses sels, en particulier en ses sels physiologiquement supportables, avec des acides ou des bases minéraux ou organiques.

**Revendications pour l'Etat contractant: AT**

1. Procédé pour la préparation de nouveaux dérivés de l'acide phénylacétique de formule générale

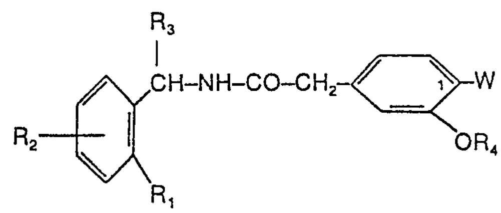

, (I)

dans laquelle

$R_1$ représente un groupe pyrrolidino, pipéridino, hexaméthylènimino, méthyl-pyrrolidino, diméthyl-pyrrolidino, 2-méthyl-pipéridino, 3-méthyl-pipéridino, 4-méthyl-pipéridino, 3,3-diméthyl-pipéridino, cis-3,5-diméthyl-pipéridino ou trans-3,5-diméthyl-pipéridino,

$R_2$ représente un atome d'hydrogène ou d'halogène, un groupe méthyle ou méthoxy,

$R_3$ représente un atome d'hydrogène, un groupe alcoyle avec 1 à 4 atomes de carbone, un groupe n-pentyle ou 3-méthyl-n-butyle ou un groupe phényle éventuellement substitué par un atome d'halogène, un groupe méthyle ou méthoxy,

$R_4$ représente un atome d'hydrogène, un groupe méthyle, éthyle ou allyle et

W représente un groupe méthyle, hydroxyméthyle, formyle, carboxy ou alcoxycarbonyle avec en tout 2 à 5 atomes de carbone, la partie alcoyle du groupe alcoxy pouvant être substituée par un groupe phényle, de leurs énantiomères et de leurs sels,

caractérisé en ce que

a) on fait réagir une amine de formule générale

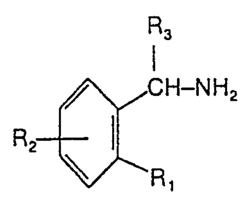

, (II)

dans laquelle

$R_1$ à $R_3$ sont définis comme au début, avec un acide carboxylique de formule générale

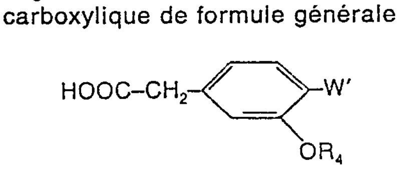

, (III)

dans laquelle

$R_4$ est défini comme au début et

W' possède les significations mentionnées au début pour W ou représente un groupe carboxyle protégé par un radical protecteur,

ou avec leurs dérivés réactifs, éventuellement préparés dans le mélange réactionnel et si nécessaire, on clive un radical protecteur utilisé, ou

b) pour la préparation d'un composé de formule générale I dans laquelle W représente un groupe carboxy, on transforme en un groupe carboxy un groupe A dans un composé de formule générale

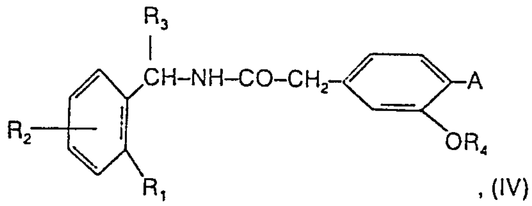

, (IV)

dans laquelle

$R_1$ à $R_4$ sont définis comme au début et

A représente un groupe transformable en un groupe carboxy par hydrolyse, thermolyse ou hydrogénolyse,

c) pour la préparation d'un composé de formule générale I dans laquelle $R_4$ représente un atome d'hydrogène, un groupe méthyle ou éthyle, on fait réagir un composé de formule générale

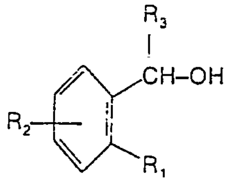

, (V)

dans laquelle

$R_1$ à $R_3$ sont définis comme au début, avec un composé de formule générale

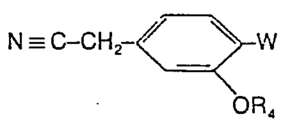

, (VI)

dans laquelle

$R_4$ et W sont définis comme au début, ou

d) pour la préparation de composés de formule générale I dans laquelle $R_4$ représente un atome d'hydrogène, on clive un radical protecteur à partir d'un composé de formule générale

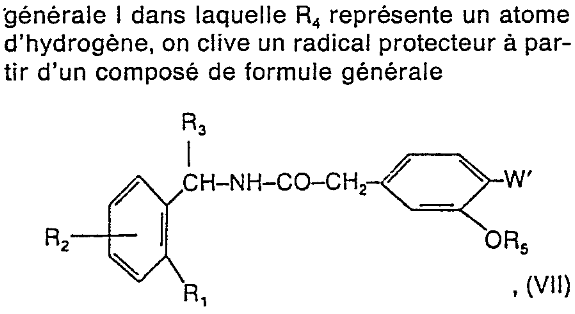

, (VII)

dans laquelle

$R_1$ à $R_3$ sont définis comme au début,

W' possède les significations mentionnées au début pour W ou représente un groupe transformable en un groupe carboxy et

$R_5$ représente un radical protecteur pour un groupe hydroxy, ou

e) pour la préparation de composés de formule générale I dans laquelle $R_4$ représente un groupe méthyle, éthyle ou allyle, on fait réagir un composé de formule générale

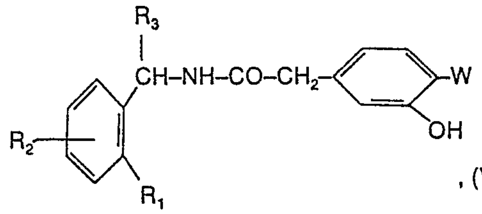

, (VIII)

dans laquelle

$R_1$ à $R_3$ et W sont définis comme au début, avec un composé de formule générale

$$R_4'-X \qquad (IX)$$

dans laquelle

$R_4'$ possède les significations mentionnées au début pour $R_4$, à l'exception de l'atome d'hydrogène et

X représente un groupe échangeable de façon nucléophile ou également ensemble avec l'atome d'hydrogène voisin, un groupe diazo, lorsque $R_4'$ représente un groupe méthyle ou éthyle, et si nécessaire on hydrolyse ensuite un composé ainsi obtenu, ou

f) un composé de formule générale

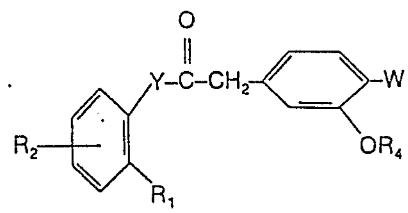

, (X)

dans laquelle

$R_1$, $R_2$, $R_4$ et W sont définis comme au début et Y représente un groupe de formule

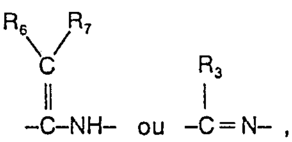

$R_3$ y étant défini comme au début et

$R_6$ et $R_7$, ensemble avec l'atome de carbone intermédiaire, y représentant un groupe alcoylidène avec 1 à 4 atomes de carbone,

est réduit, et

si on le désire, on transforme ensuite un composé ainsi obtenu de formule générale I dans laquelle $R_2$ représente un atome d'halogène et/ou $R_3$ représente un groupe halogénophényle et/ou W représente un groupe hydroxyméthyle qui a été transformé en un groupe halogénométhyle, au moyen d'une déshalogénation en un composé correspondant de formule générale I dans laquelle $R_2$ représente un atome d'hydrogène et/ou $R_3$ représente un groupe phényle et/ou W représente le groupe méthyle, et/ou

on transforme un composé ainsi obtenu de formule générale I dans laquelle W représente le groupe carboxy, par estérification en un composé

correspondant de formule générale I dans laquelle W représente un groupe alcoxycarbonyle ou phénylalcoxycarbonyle, et/ou

on transforme un composé ainsi obtenu de formule générale I dans laquelle W représente un groupe carboxy, alcoxycarbonyle ou phénylalcoxycarbonyle, par réduction en un composé correspondant de formule générale I dans laquelle W représente un groupe formyle ou hydroxyméthyle, et/ou

on transforme un composé ainsi obtenu de formule générale I dans laquelle W représente le groupe hydroxyméthyle, par oxydation en un composé correspondant de formule générale I, dans laquelle W représente un groupe formyle ou carboxy, et/ou

on transforme un composé ainsi obtenu de formule générale I dans laquelle W représente le groupe carboxy, par transformation en un hydrazide d'acide sulfonique et dismutation subséquente en un composé correspondant de formule générale I, dans laquelle W représente le groupe formyle et/ou

on sépare un composé racémique ainsi obtenu de formule générale I dans laquelle $R_3$ est défini comme au début, à l'exception de l'atome d'hydrogène, par chromatographie sur des phases chirales en ses énantiomères, et/ou

on transforme un composé ainsi obtenu de formule générale I en ses sels, en particulier en ses sels physiologiquement supportables, avec des acides ou des bases minéraux ou organiques.

2. Procédé selon la revendication 1, caractérisé en ce que la réaction est effectuée dans un solvant.

3. Procédé selon les revendications 1a et 2, caractérisé en ce que la réaction est effectuée en présence d'un agent activant les acides, d'un agent d'élimination de l'eau ou en présence d'un agent activant l'amine.

4. Procédé selon les revendications 1a et 3, caractérisé en ce que la réaction est effectuée à des températures entre −25 et 250 °C, de préférence à des températures entre −10 °C et la température d'ébullition du solvant utilisé.

5. Procédé selon les revendications 1b et 2, caractérisé en ce que lorsque A représente le groupe nitrile ou aminocarbonyle, la réaction est effectuée en présence d'un nitrite, par exemple le nitrite de sodium, et en présence d'un acide tel que l'acide sulfurique à des températures entre 0 et 50 °C ou au moyen d'acide phosphorique à 100% à des températures entre 100 et 180 °C, de préférence à des températures entre 120 et 160 °C.

6. Procédé selon les revendications 1c et 2, caractérisé en ce que la réaction est effectuée à des températures entre 0 et 150 °C, de préférence à des températures entre 20 et 100 °C.

7. Procédé selon les revendications 1d et 2, caractérisé en ce que la réaction est effectuée en présence d'un acide à des températures entre 0 et 150 °C ou au moyen de tribromure de bore à des températures entre −78 et 20 °C.

8. Procédé selon les revendications 1e et 2, caractérisé en ce que la réaction est effectuée à des températures entre 0 et 100 °C, de préférence à des températures entre 20 et 50 °C.

9. Procédé selon les revendications 1f et 2, caractérisé en ce que la réduction est effectuée au moyen d'hydrogène, en présence d'un catalyseur d'hydrogénation à des températures entre 0 et 100 °C, de préférence toutefois à des températures entre 20 et 50 °C.

10. Procédé pour la préparation d'un médicament pour le traitement du diabète mellitus, caractérisé en ce qu'on introduit un composé de formule générale I selon la revendication 1, dans laquelle $R_4$ représente un groupe méthyle, éthyle ou allyle, ou un sel physiologiquement supportable de celui-ci, dans un ou plusieurs excipients et/ou diluants inertes.